# FASCICULE DE BREVET EUROPEEN

(11) **EP 0 457 701 B1**
(45) Date de publication et mention de la délivrance du brevet: **22.02.1995**
(21) Numéro de dépôt: 91430009.0
(22) Date de dépôt: 13.05.1991
(51) Int. Cl.: C07C 229/36, C07D 209/16, G01N 33/78

(54) **Nouveaux dérivés de médiateurs endogènes, leurs sels, procédé de préparation, applications, et compositions les renfermant**
Derivate von endogenen Mediatoren, ihre Salze, Verfahren zur Herstellung, Anwendungen und Zusammensetzungen, die diese enthalten
Derivatives of endogenous mediators, their salts, process for preparation, application and compositions containing them

(30) Priorité: 15.05.1990 FR 9006292; 30.01.1991 FR 9101292
(43) Date de publication de la demande: 21.11.1991
(73) Titulaire: IMMUNOTECH S.A., F-13276 Marseille Cédex 9 (FR)
(72) Inventeur: Chauveau, Jacques, F-13008 Marseille (FR); Delaage, Michel, F-13001 Marseille (FR); Morel, Anne, F-13008 Marseille (FR); Segu, Louis, F-13400 Marseille (FR)
(74) Mandataire: Rinuy, Santarelli

(56) Documents cités:
- EP-A- 0 006 792
- DE-A- 2 737 802
- US-A- 4 040 907
- JOURNAL OF BIOLOGICAL CHEMISTRY, vol. 261, no. 25, 5 septembre 1986, pages 11613-11622, Baltimore, US; J. KOEHRLE et al.: "Rat liver iodothyronine monodeiodinase"
- EUROPEAN JOURNAL OF PHARMACOLOGY, vol. 194, 1991, pages 91-98; P. BOULENGUEZ etal.: "A new 5-hydroxy-indole derivative with preferential affinity for 5-HT1B binding sites"

## Description

La présente invention concerne de nouveaux dérivés de médiateurs endogènes, leurs sels, leur procédé de préparation et leurs applications notamment à l'analyse des médiateurs endogènes, l'analyse ou la purification des récepteurs desdits médiateurs, la visualisation de leurs sites récepteurs, la préparation d'anticorps et l'utilisation desdits anticorps pour l'analyse des médiateurs endogènes, leur application à titre de médicaments et compositions les renfermant.

Les messages entre les cellules sont transmis grâce à des vecteurs chimiques (hormone ou neuromédiateur) dont la spécificité est assurée par la protéine cible : le récepteur. Les molécules porteuses de l'information peuvent être des peptides ou des molécules de faible poids moléculaire. Parmi celles-ci, un certain nombre présentent simultanément une ou plusieurs fonctions hydroxyle et une ou plusieurs fonctions amine, par exemple les indolamines, les catécholamines (neuromédiateurs) et la thyroxine (hormone).

Ces molécules jouent un rôle primordial dans la physiologie : les indolamines et les catécholamines dans la transmission et l'intégration de l'information dans le système nerveux central et la thyroxine dans la régulation du métabolisme basal. Il est essentiel d'en assurer le dosage de façon à affiner les diagnostics ou à aider les recherches fonctionnelles. Dans le cas des neuromédiateurs, les sites de liaison des récepteurs assurent la spécificité de la réponse de la cellule effectrice : la modification chimique ponctuelle des ligands endogènes permet de différencier les divers types et sous-types de récepteurs membranaires.

Les ligands modifiés peuvent être des drogues agissant spécifiquement sur les fonctions physiologiques contrôlées par ces récepteurs.

Certaines hormones sont présentes au niveau sérique sous forme liée à des protéines porteuses. Ces protéines assurent le transport de ces hormones du lieu de synthèse aux cellules-cibles. Les hormones thyroïdiennes sont présentes sous forme non liée à des taux inférieurs à 1 % de la quantité totale d'hormones (libre + liée). La modification chimique ponctuelle des hormones thyroïdiennes permet d'améliorer leur dosage.

Les Demandeurs ont découvert que l'on peut atteindre ces objectifs grâce à une O-carboxyméthylation sur le groupement hydroxyphényle des molécules endogènes portant également une amine primaire.

La carboxyméthylation (Gurd, Methods in Enzymology, vol. XI, 1967, p. 532-541) a souvent été utilisée pour bloquer les fonctions amines des amino acides. La carboxyméthylation des groupements hydroxyphényle est décrite comme une réaction parasite (Korman et Clarke, J. Biol. Chem. 221, 1956, p. 113-131). Celle des groupements hydroxyphényle (Spector, 1982) a été utilisée pour coupler la morphine (ne possédant pas de fonction amine) à une protéine en vue de l'obtention d'anticorps.

C'est pourquoi la présente demande a pour objet de nouveaux dérivés de molécules biologiquement actives comportant une fonction amine primaire et un noyau hydroxylé ainsi que leurs sels d'addition avec les acides minéraux ou organiques, caractérisés en ce qu'ils répondent à la formule générale I :

[R'R''N-A-B-O-CH₂-CO]ₙR₁ (I)

dans laquelle
n représente un nombre entier de 1 à 10 ;
A représente une chaîne alcoylène linéaire ou ramifiée renfermant de 1 à 5 atomes de carbone ;
B représente un noyau aromatique comportant de 6 à 10 atomes de carbone, le cas échéant substitués et comportant éventuellement un hétéroatome,
- R₁ représente un reste aminé, un reste alcool choisi parmi les phénols éventuellement substitués et les alcools aliphatiques en C₁-C₁₆,
et
R' et R'' représentent un radical alcoyle renfermant de 1 à 5 atomes de carbone ou un atome d'hydrogène, ainsi que leurs sels d'addition avec les acides minéraux ou organiques.

Les sels d'addition avec les acides minéraux ou organiques peuvent être par exemple des sels formés avec les acides chlorhydrique, bromhydrique, nitrique, sulfurique, phosphorique, acétique, formique, propionique, benzoïque, maléique, fumarique, succinique, tartrique, citrique, oxalique, glyoxylique, aspartique, alcane sulfoniques tels que les acides méthane ou éthane sulfoniques, arylsulfoniques, tels que les acides benzène ou paratoluène sulfoniques ou carboxyliques. On retient plus particulièrement les sels d'anions chaotropiques, propres à faciliter la dissolution en milieu aqueux, tels que les ions citrates ou succinates.

La chaîne alcoylène représentée par A peut être une chaîne propylène, isopropylène, méthylène, et de préférence éthylène ; elle peut être substituée par un groupement sulfhydrile.

Le noyau aromatique peut comporter un seul cycle tel qu'un noyau phényle ou deux cycles, comme par exemple un noyau indole. Un ou plusieurs des atomes de carbone de B peuvent être substitués par un radical choisi parmi les atomes d'halogène tel que chlore, ou des radicaux alcoxy tels qu'éthoxy ou de préférence méthoxy, alkyle tels que propyle, éthyle, de préférence méthyle, alkylthio tel que méthylthio, polyhalogénoalkyle tel que trifluorométhyle.

L'hydroxyle substitué par le radical -CH₂-CO-NH-R selon la présente invention peut se trouver en toutes positions mais se trouve notamment sur un cycle phényle du noyau et de préférence dans les positions que ce radical hydroxyle occupe habituellement dans les médiateurs naturels.

Lorsque R' et R'' représentent un radical alcoyle, il s'agit de préférence d'un radical méthyle ou éthyle. R' et R'' représentent avantageusement l'hydrogène. Par radical aminoacyle l'on entend des restes d'amino acides, peptides ou protéines. Par radical hydrophobe l'on entend que ledit radical possède des chaines saturées et ne comporte pas de groupements tels que amino ou hydroxy libres.

R' et R'' représentent avantageusement un atome d'hydrogène, un radical méthyl, éthyl, le radical NR'R'' pouvant éventuellement être quaternisé et on peut citer par exemple les radicaux triméthyl ammonium ou diéthylméthyl ammonium.

R' et R'' représentent également notamment un radical tyrosyl ou lysyl.

Dans le cas où R₁ représente un reste aminé, symbolisé par -NH-R, le reste R qui est donc fixé au carboxyle par une liaison amide peut être de toute nature mais est de préférence un reste apte au marquage, par exemple à l'aide d'un ou plusieurs atomes radioactifs, tel que l'¹²⁵I.

La nature chimique de ce reste comprendra de préférence un acide aminé qui servira à la liaison avec le reste de la molécule de formule (I). Ce reste aminé sera par exemple une protéine ou un polypeptide renfermant de 2 à 10 acides aminés et de préférence 2 ou 3 acides aminés, ou encore un acide monoaminé, tel la tyrosine, ou diaminé.

Lesdits acides aminés seront de préférence les acides aminés naturels ou leurs dérivés amidés ; c'est ainsi par exemple que si le radical R₁ est un dipeptide constitué d'un reste glycyle et d'un reste tyrosyle, on pourra par exemple remplacer le reste tyrosyle par un reste tyrosynamide.

La diamine pourra servir au couplage de fluorophores ou à la synthèse de dimères de formule (I') :

R'R'' N-A-B-O-CH₂-CO-NH-R-NH-CO-CH₂-O-B-A-N-R'R'' (I')

dans laquelle R, R', R'', A et B ont la signification déjà indiquée.

Parmi les restes aminés, on peut citer notamment les restes amino, hydrazino, nitrilo (-N₃), les peptides ou dérivés de peptides suivants : tyrosyl cystéine, tyrosyl-cystéinamide, tyrosyl glycine, tyrosyl glycinamide.

On peut citer également les dérivés protéiques reliés au carboxyle directement ou par l'intermédiaire d'un peptide ou d'un aminoacide et d'un agent hétérobifonctionnel susceptible de réagir avec un groupe sulfhydrile et un groupe amino, tel le SMCC, et on peut citer par exemple les restes tyrosyl-cystéinyl-protéine, tyrosyl-cystéinyl-agent hétérobifonctionnel tel (le SMCC)-protéine, glycyl tyrosine, ou le reste glycyl-1,4-butanediamine.

Parmi les restes dérivés d'un alcool, on peut citer notamment ceux dérivés de phénols, éventuellement substitués sur le phényl, tel le 2 ou 4-nitrophénol, ceux dérivés du phényl méthanol, éventuellement substitués sur le phényl par un radical alkyle, ceux dérivés d'hydroxyalkyl triméthylsilyle notamment l'hydroxy éthyl, ceux dérivés des alcools aliphatiques en C₁-C₁₆.

Des dérivés préférés selon l'invention sont des dérivés de formule (I) tels que définis ci-dessus, caractérisés en ce que B représente un noyau phényle, éventuellement substitué par un ou plusieurs atomes d'halogène, ou un noyau indole, ainsi que leurs sels d'addition avec les acides minéraux ou organiques. Les noyaux peuvent être substitués par un atome d'halogène, de préférence le chlore, le brome ou l'iode, notamment le noyau indole, et dans ce cas, de préférence en position 2.

Parmi ces derniers, pour lesquels B représente un noyau indole, on retient notamment ceux caractérisés en ce que R₁ représente une diamine, une protéine, un acide aminé ou un polypeptide constitué au plus de 5 acides aminés ou des dérivés desdits acides aminés ou polypeptides, et notamment le tryptamine 5-O-carboxyméthylglycyltyrosinamide [S-CM-GTNH₂], de même que les dérivés cités dans les exemples, ainsi que leurs sels d'addition avec les acides minéraux ou organiques.

Parmi ceux-ci, on retient également ceux caractérisés en ce que A représente un radical -(CH₂)₂- et R' et R'' représentent un radical alkyle linéaire ou ramifié renfermant de 1 à 5 atomes de carbone.

La présente demande a aussi pour objet un procédé de préparation des dérivés ci-dessus décrits, caractérisé en ce que l'on fait réagir un dérivé de formule (II) :

R'R'' N-A-B-OH (II)

dans laquelle R', R'', A et B ont la signification déjà indiquée, avec un dérivé comportant un groupement protecteur des fonctions amine dans le cas où R'=R''=H, pour obtenir un dérivé de formule (III) :

Y-HN-A-B-OH (III)

dans laquelle A et B ont la signification déjà indiquée et Y représente un groupement protecteur des fonctions amine, facilement clivable, que l'on fait réagir avec un acide halogéno-acétique pour obtenir un dérivé de formule (IV) :

Y-HN-A-B-O-CH₂COOH (IV)

dans laquelle A, B et Y ont la signification déjà indiquée, que l'on fait réagir avec un dérivé aminé ou un alcool pour obtenir le dérivé de formule (I) telle que définie ci-dessus le cas échéant sous forme dimère de formule (I') :

R'R'' N-A-B-O-CH₂-CO-NH-R-NH-CO-CH₂-O-B-A-N-R'R'' (I')

dans le cas d'une diamine, ou polymère dans le cas d'une polyamine que l'on isole et, si désiré, salifie.

Les dérivés de formule générale I présentent un caractère basique. On peut avantageusement préparer le cas échéant des sels des dérivés de formule générale I, en faisant réagir en proportions sensiblement stoechiométriques un acide minéral ou organique avec ledit dérivé de formule générale I. Les sels peuvent éventuellement être préparés sans isoler les bases correspondantes.

Le dérivé apte à greffer un groupement protecteur des fonctions amine sur le dérivé de formule (II) peut être par exemple un chlorure d'acide [9-fluorphenylméthyl oxycarbonyl (FmocCl), benzyloxycarbonyl (BzCl)] et de préférence un anhydride [le di-t-butyldicarbonate, (BOC)₂O, ou l'anhydride citraconique].

La préparation du dérivé de formule (IV) est réalisée de préférence à pH alcalin en présence d'un oxyde métallique tel que l'oxyde de magnésium et d'un acide halogéno-acétique, ce dernier pouvant être un chlorure mais étant de préférence un bromure.

La réaction du dérivé de formule (IV) avec le dérivé aminé (R₁) est réalisée de préférence après activation du groupement carboxylique sous forme d'anhydride mixte par un alkylchlorocarbonate tel que l'éthylchloroformate. On peut utiliser également comme activateurs les chlorures d'acide, les carbodiimides ou la formation préalable d'un ester hydrolysable de N-hydroxysuccinimide.

Le clivage du groupement protecteur de l'amine est réalisé si nécessaire par hydrolyse acide, notamment à l'aide d'un acide tel que l'acide trifluoroacétique, ou alkaline, notamment à l'aide de la pipéridine.

Les médiateurs de formule (II) et leurs analogues de synthèse sont bien connus par diverses publications et brevets.

Le couplage du dérivé de formule (I), ou de l'un de ses sels, à un marqueur peut être réalisé grâce à la présence d'une fonction carboxyle libre dans le résidu R₁ de la formule I, tel que décrit pour la liaison du reste aminé au reste de la molécule de formule I.

Si le marqueur est l'iode, le reste aminé comportera une tyrosine (ou une tyrosinamide) ou une histamine.

Si le marqueur est une enzyme, le reste aminé comportera des groupements carboxyle ou sulfhydrile.

Si le marqueur est un élément fluorescent, le reste aminé comportera une diamine.

On retient encore les dérivés de formule I ci-dessus décrits, caractérisés en ce qu'ils comportent une protéine greffée sur le chaînon O-carboxyméthyle selon les procédés ci-dessus.

Les dérivés objet de la présente invention possèdent de très intéressantes propriétés pharmacologiques. Ils sont doués notamment d'une remarquable affinité pour les récepteurs de la sérotonine particulièrement 5HT_{1D}.

Ces propriétés sont illustrées plus loin dans la partie expérimentale. Elles justifient l'utilisation des dérivés de médiateurs ci-dessus décrits ainsi que de leurs sels d'addition avec les acides pharmaceutiquement acceptables à titre de médicament.

Les médicaments selon la présente invention trouvent leur emploi par exemple dans le traitement tant curatif que préventif des maladies liées à un dysfonctionnement des récepteurs 5HT et particulièrement des différents récepteurs 5HT_{1D}, à leur dérégulation ou à des modifications du ligand endogène (généralement la sérotonine). Ils trouvent en particulier leur emploi dans le traitement de la migraine.

La dose usuelle, variable selon le sujet traité et l'affection en cause, peut être, par exemple, de 0,1 à 10 mg par jour par voie orale chez l'homme du dérivé de l'exemple 1.

L'invention a aussi pour objet les compositions pharmaceutiques qui renferment au moins un dérivé précité ou un de ses sels d'addition avec les acides pharmaceutiquement acceptables, à titre de principe actif.

A titre de médicaments les dérivés répondant à la formule générale I ainsi que leurs sels d'addition avec les acides pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme par exemple les comprimés simples ou dragéifiés, les gélules, les granulés, les caramels, les suppositoires, les préparations pour instillations nasales, les préparations injectables ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Les dérivés de formule (I) possèdent, comme on l'a vu, une importante affinité pour les récepteurs ou également la protéine de transport de la molécule endogène correspondante.

Les dérivés objet de la présente demande éventuellement marqués par exemple à l'aide d'un élément radioactif, notamment l'¹²⁵I, ou fluorescent ou encore à l'aide d'une enzyme sont utiles notamment pour l'imagerie tant in vitro que pour certains in vivo des sites de liaison des médiateurs endogènes. Ces propriétés sont illustrées ci-après dans la partie expérimentale. On peut aussi utiliser comme marqueur une protéine portant un or colloïdal ou un produit de contraste.

La présente demande a donc également pour objet les dérivés ci-dessus décrits, caractérisés en ce qu'ils sont sous une forme marquée.

La présente demande a aussi pour objet l'application des dérivés ci-dessus décrits à la purification des récepteurs des médiateurs endogènes.

La O-carboxyméthylation des hormones thyroïdiennes module l'interaction de ces hormones avec leurs protéines porteuses. Cette propriété peut être utilisée pour éliminer la liaison de ces hormones thyroïdiennes natives à ces protéines porteuses. Dans le cadre d'une activité immunoanalytique, la création d'analogues non reconnus par l'anticorps, inhibiteurs de la liaison aux protéines de transport, permet le dosage de l'hormone totale.

La présente demande a encore pour objet l'application des dérivés ci-dessus décrits et en particulier les dérivés de la thyroxine O-carboxyméthyle à l'inhibition de la liaison aux protéines de transport, pour le dosage de l'hormone totale.

Les dérivés de la présente demande, pour lesquels R₁ est une protéine, permettent la préparation d'anticorps dirigés contre le médiateur correspondant au reste de la molécule de formule (I).

La présente demande a également pour objet l'application des dérivés ci-dessus décrits à l'imagerie des sites de liaison des médiateurs endogènes.

La présente demande a tout autant pour objet l'application des dérivés de formule (I) à la préparation d'anticorps dirigés contre les médiateurs endogènes.

Le dosage des médiateurs endogènes [de formule (II), R'R''-N-A-B-OH] ou de leurs dérivés pourra être assuré grâce à ces anticorps, en utilisant les dérivés de formule (I) comme traceur.

La présente demande a enfin pour objet les kits d'analyse, caractérisés en ce qu'ils renferment l'un au moins des dérivés de formule (I) ci-dessus décrits.

Les exemples qui suivent illustrent la présente invention sans toutefois la limiter.

### PARTIE EXPERIMENTALE

### PREPARATION 1. Synthèse de dérivés de la thyroxine

Série d'exemples répondant à la formule générale I :

R'R''-N-A-B-O-CH₂-CO-R₁ (I)

Dans laquelle R' = R'' = H A = - CH(COOH)-CH₂ B est du type -B₁-X-B₂- avec X = Oxygène si B₁ = B₂ = C₆H₂I₂ ; dérivés de la 3,3',5,5'-tétraiodo-L-thyronine ou L-thyroxine (T4)
si B₁ = C₆H₂I₂ et B₂ = C₆H₃I dérivés de la 3,3',5-triiodo-L-thyronine (T3)

### Stade A

### 1. Synthèse du N-tertiobutyl carbamate-O-carboxyméthyl T4 (BOC-T₄-O-CH₂-COOH) ou T₃(BOC-T₃-O-CH₂-COOH)

### 1.1. Protection du groupe amine

Pour les synthèses de l'exemple 1, on utilise la T₄ ou de T₃ sous forme d'acide libre ou modifié par exemple par amidation.

La protection du groupe amine de la T4 et de la T3 est réalisée avec le di-t-butyl dicarbonate [(BOC)₂O] (Tarbell et al., Proc. Nat. Acad. Sci. USA, 69, 1972, 730-732). A 10 »moles de T₄ ou de T₃ sont ajoutés 30 »l de triéthylamine (TEA, 7,2 N) et 240 »l de (BOC)₂O 50 mM dans le diméthyl sulfoxide (DMSO).

### 1.2 Carboxyméthylation du groupement hydroxyphényle

La solution obtenue précédemment est mélangée volume à volume avec une solution aqueuse d'acide bromoacétique 500 mM, le pH ajusté à 12, en présence d'oxyde de magnésium et d'azote gazeux. L'agitation est maintenue pendant 24 h à l'obscurité. Le milieu est centrifugé 10 minutes à 10 000 tours/minute.

### 1.3 Purification des produits par chromatographie liquide à haute performance (CLHP).

Le surnageant est dilué dans 5 volumes d'acide trifluoroacétique (TFA) 0,05 %. Un ml de ce mélange est injecté dans une colonne de silice greffée » Bondapack C ₁₈ (10 »m, diamètre 3,9 mm, longueur 30 cm). Les dérivés de la T3 sont élués en isocratique pendant 30 mn avec un mélange TFA 0,05 % 50 vol. - méthanol 50 vol. ; puis avec un gradient atteignant 100 % de méthanol en 60 minutes. Pour les dérivés de la T4, le mélange initial d'élution isocratique est composé de TFA 0,05 % 40 vol. - méthanol 60 vol. Le débit est de 1 ml/mn.

Les produits initiaux (T3, T4) sont recueillis au cours de la période isocratique. Les dérivés substitués sont recueillis dans le gradient :
BOC-T₃ : 75 % méthanol
BOC-T₃-O-CH₂-COOH : 78 % méthanol
BOC-T₄ : 80 % méthanol
BOC-T₄-O-CH₂-COOH : 82 % méthanol

### (Figure 1)

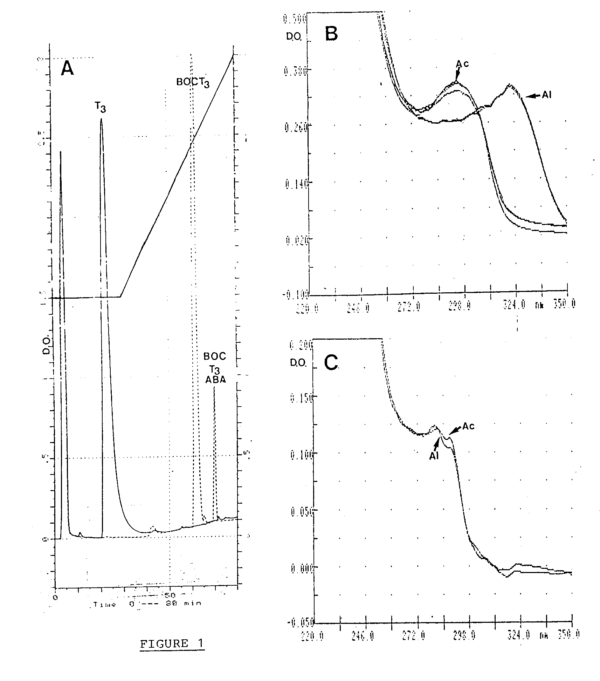

Les fractions recueillies sont analysées en spectrophotométrie UV. Les spectres d'absorption en milieu basique et acide sont comparés de façon à déterminer si la substitution a bien eu lieu sur le groupement hydroxyle (Korman Clarke, op. cité). Les dérivés O-carboxyméthylés ne présentent pas de déplacement de spectre en milieu basique (Figure 1). Les fractions contenant le dérivé BOC-T₃-O-CH₂-COOH et BOC-T4-O-CH2-COOH sont évaporées et lyophilisées.

### 2. Conjugaison des dérivés O-carboxyméthylés avec le radical R-NH₂

### 2.1 Allongement de la chaîne latérale nouvellement créée :

On peut créer une liaison amide entre le groupement carboxyméthyle et un amino-acide, une chaîne peptidique ou une protéine native ou modifiée.

Le groupement carboxyle du BOC-T3-O-CH2-COOH ou BOC-T4-O-CH2-COOH est activé par l'éthylchloroformate (ECF,7 »l additionné de 7 »l de TEA dans 5 ml de diméthylformamide). Un volume de cette solution tel que l'ECF soit équimolaire avec le produit à activer est versé sur le lyophylisat. Après 5 mn d'activation à 4°C, on ajoute un volume égal de solution aqueuse de NH₂-R à une concentration 50 fois supérieure à celle du dérivé carboxyméthylé. NH₂-R peut être l'histamine ou Gly-Tyr, Tyr-Gly, Cys, Gly-Cys, sous forme d'acide libre ou d'amide.

Les produits sont séparés par CLHP sur colonne » Bondapack C18 en gradient TFA 0,05 % - Méthanol.

### 2.2 Préparation de dérivés macromoléculaires

### 2.2.1. Greffe des dérivés BOC-T3-O-CH2-CO-NH-R sur des protéines.

Les dérivés obtenus en 1.3. ou 2.1. ci-dessus ou sous forme acide libre, sont activés par l'éthylchloroformate (voir 2.1.) et greffés sur une protéine native (BSA, sérum albumine de boeuf) ou modifiée (Gly-BSA).

On sépare par dialyse les dérivés BOC-T3-O-CH₂-CO-NHR des dérivés couplés à la BSA, du type BOC-T₃-O-CH₂-CO-NH-R-BSA avec
R = His, Gly-Tyr, Tyr-Gly, Cys, Gly-Cys
Le groupement amine de ces dérivés peut être déprotégé selon 3.

### 2.2.2. Greffe des dérivés T3-O-CH2-CONH-Cys ou T₃-O-CH₂-CONH-Gly-Cys sur des protéines.

Des protéines sont modifiées par adjonction d'un agent de réticulation hétérobifonctionnel du type N-hydroxysuccinimide, par exemple le succinimidyl-4-(N-maléimidométhyl)cyclohexane-1-carboxylate (SMCC) la réaction (Ishikawa et al., 1983, J. Immunoassay 4, 209-327) a lieu à pH 7,3 dans le tampon phosphate. La protéine dont les résidus NH₂ sont ainsi activés est séparée par dialyse.

Les dérivés T₃-O-CH₂-CONH-Cys ou T₃-O-CH₂-CONH-Gly-Cys (amidés) sont conjugués par le groupe sulfhydrile aux protéines modifiées, à pH 6,5.

Les produits sont séparés par dialyse, on obtient des dérivés de formule générale T₃-O-CH₂-CO-NH-Cys-S-SMCC-BSA.

### 3. Préparation du O-carboxyméthyl thyroxine (T₄-O-CH₂-COOH)

On procède à la déprotection du groupement amine par adjonction de 200 »l de TFA (200 mg) au dérivé BOC-T₃-O-CH₂-COOH ou BOC-T₄-O-CH₂-COOH lyophilisés et refroidis à -20°C. Après une minute d'action, le TFA est évaporé sous azote gazeux.

### Stade B

### 4. Iodation des dérivés

On ajoute à 1 nmole de T₃, de BOC-T₃ ou de BOC-T₃-O-CH₂-COOH, 1 mCi de [¹²⁵I]Na (2000 Ci/mmole, NEN) et 10»l de chloramine T (CT 1mg/ml). Après 90 sec., la réaction est arrêtée par 50 »l de métabisulfite de sodium (MBS) pendant 2 mn. Après adjonction de 20 »l de méthanol et agitation, on dilue dans 1,5 ml de TFA (0,05 %).

La séparation des produits de la réaction se fait selon le protocole décrit en A.1. pour les dérivés de l'exemple 1. Les produits sont exclus dans le gradient méthanol respectivement à : [¹²⁵I]T₄ 62 % ; BOC [¹²⁵I]T₄, 80 % ; BOC[¹²⁵I]T₄-OCH₂COOH, 82 %.

Les dérivés radiomarqués sont dilués dans le méthanol. Le BOC [¹²⁵I]T₄-O-CH₂-COOH est déprotégé comme indiqué en 3.

### EXEMPLE 1. Synthèse de dérivés de la sérotonine

Série d'exemples répondant à la formule générale I :
R'R''-N-A-B-O-CH₂-CO-R₁, dans laquelle R' = R'' = H
A = -CH₂-CH₂-
B = noyau indole (OH en 5, aminoéthyl en 3)

### 1. Synthèse du N-tertiobutyl carbamate, 5-O-carboxyméthyl tryptamine [BOC-S-CM]

Dérivé de formule type (IV) Y-HN-A-B-O-CH₂-COOH

### Stade A

### 1.1. Protection du groupe amine

La protection du groupement amine de la 5-HT est réalisée avec le di-t-butyldicarbonate [(BOC)₂O] (Tarbell et al., 1972). On procède au mélange de volumes égaux d'oxalate de sérotonine 50 mM et de (BOC)₂0 50 mM dans le diméthylsulfoxyde (DMSO) en présence de triéthylamine (TEA). La réaction est immédiate à température ambiante.

### 1.2. Carboxyméthylation du groupement hydroxyphényle

La protection par le BOC n'est pas utile pour certains dérivés de la sérotonine qui, comme la bufoténine, possèdent une amine tertiaire, les résidus R' et R'' de la formule générale étant des groupes méthyle. Le reste de la molécule (résidus A et B) étant identiques à la sérotonine, on peut procéder directement à la O-carboxyméthylation de la bufoténine dans les mêmes conditions que celles décrites ci-après pour le BOC-S.

La solution obtenue en 1.1. ci-dessus (exemple 2) est mélangée volume à volume avec une solution aqueuse d'acide bromoacétique 500 mM, le pH ajusté à 12 en présence d'oxyde de magnésium et d'azote gazeux. Le pH, l'absence d'oxygène et de lumière sont maintenus constants pendant 24 heures. Le milieu est centrifugé 5 mn à 10 000 tours/mn.

### 1.3. Purification par chromatographie liquide à haute performance (CLHP)

La séparation du dérivé carboxyméthyl-O-5-tryptamine-BOC (BOC-S-CM) de la sérotonine BOC (BOC-S) et des produits d'oxydation se fait par CLHP.

Le surnageant est dilué dans deux volumes d'une solution aqueuse à 1 % d'acide trifluoroacétique. 1,8 ml de ce mélange est injecté dans un CLHP sur colonne de silice greffée Ultrasphère ODS C₁₈ (5 »m, diamètre 4,6 mm, longueur 15 cm), par élution isocratique (H₂O-TFA 0,05 %, 55 vol, méthanol 45 vol). L'élution est suivie par spectrophotométrie.

La séparation des produits de carboxyméthylation de la bufoténine a lieu sur colonne » Bondapack C₁₈, en élution isocratique TFA 0,05 % 93 vol, acétonitrile 7 vol.

Les fractions recueillies sont analysées en spectrophotométrie. Les spectres d'absorption en milieu basique et acide sont comparés de façon à déterminer si la substitution a bien eu lieu sur le groupement hydroxyle (Korman Clarke, op. cité).

Les fractions contenant le dérivé BOC-S-CM sont évaporées et lyophilisées.

### 2. Conjugaison de dérivés O-carboxyméthylés avec le radical R-NH₂.

### 2.1. Allongement de la chaîne latérale nouvellement créée.

On peut créer une liaison amide avec une chaîne peptidique après activation du groupement acide du BOC-S-CM par l'éthylchloroformate (ECF).

La solution d'activation est composée de 5 ml de diméthylformamide (DMF), 7 »l de TEA et 7 »l d'ECF. Un volume de cette solution tel que l'ECF soit équimolaire avec le BOC-S-CM est versé sur le dérivé BOC-S-CM lyophilisé.

Après 5 minutes d'activation à 4°C, on ajoute un volume égal de solution aqueuse de glycyl-tyrosinamide (ou d'histamine, ou de Tyr-Gly, Gly-Tyr, Gly-Gly, Gly-Cys sous forme d'acides libres ou amidés) à une concentration 50 fois supérieure à celle du BOC-S-CM.

Les produits de la réaction sont séparés par CLHP sur colonne Ultrasphère ODS C₁₈, par élution isocratique (H₂0, pH 6 60 vol, méthanol 40 vol ).

Les fractions contenant le dérivé sérotoninepeptide (BOC-S-CM-GTNH2) sont évaporées et lyophilisées.

### 2.2. Préparation de dérivés macromoléculaires

### 2.2.1. Greffe de dérivés BOC-S-CM-R sur protéine

Les dérivés obtenus en 1.3. ou 2.1. ci-dessus ou sous forme acide libre sont activés par l'éthylchloroformate (voir 2.1.) et greffés sur une protéine native (BSA) ou modifiée (Gly-BSA).

On sépare par dialyse les dérivés BOC-S-CM-R des dérivés couplés à la BSA du type BOC-S-CM-R-BSA.

Le groupement amine de ces dérivés peut être déprotégé selon 3 ci-dessus.

### 2.2.2. Greffe de dérivés S-CM-Gly-Cys sur des protéines.

Les protéines sont modifiées par adjonction d'agents hétérobifonctionnels (voir 2.2.2. de l'exemple 1).

Le dérivés S-CM-Gly-Cys est greffé sur les protéines précédentes par le groupe sulfhydryle.

Les produits sont séparés par dialyse, on obtient des dérivés de type S-CM-Gly-Cys-S-SMCC-BSA.

### 3. Déprotection du groupe amine

On verse 200 »l de TFA (200 mg) sur le dérivé de type BOC-S-CO-NHR (avec R = H, Gly-TyrNH₂, His, Gly-Tyr OH, Tyr-GlyNH₂, Gly-GlyOH, Gly-Cys) lyophilisé et refroidi à -20°C. Après une minute d'action, le TFA est évaporé sous azote gazeux.

Les produits de la réaction sont séparés par CLHP sur colonne Ultrasphère ODS C₁₈, par élution isocratique (TFA 0,05 % dans l'eau 65 vol, méthanol 35 vol).

Les fractions contenant le dérivé présentant le groupement amine libre (S-CM-GTNH₂) sont évaporées et lyophilisées.

### Stade B

### 4. Iodation des dérivés S-CM-R

Lorsque le radical R comporte une histamine ou une tyrosine, il est possible de procéder à une iodation par la chloramine T.

On ajoute à 10 »l de S-CM-R (1nmole en milieu PBS), 1mCi de ¹²⁵INa (2000 Ci/mmole, NEN) et 20 »l de CT (1 mg/ml). Au bout de 90 secondes, la réaction est arrêtée par 50 »l de MBS 50 mM. On dilue dans 1,6 ml de TFA 1 %.

La séparation des produits de la réaction a lieu par CLHP sur colonne » Bondapack C₁₈ par élution isocratique (TFA 0,05 % dans l'eau 72 vol, méthanol 28 vol).

Les fractions obtenues sont comptées en spectrométrie gamma et diluées dans une solution de Krebs.

### I Caractéristiques de la liaison des dérivés thyroxine sur les "binding protéines" de la thyroxine.

Du sérum humain normal (100»l) est additionné des dérivés du stade B de la préparation 1 (0,1 »l). On précipite par 500 »l de charbon dextran pendant 5 mn à 4°C. Après centrifugation, 10 mn à 1000 tours/mn, on compte l'activité du culot.

La liaison persistant par rapport à la liaison initiale est de 99,9 % pour la [¹²⁵I]T₄, de 80 % pour le BOC[¹²⁵I]-T₄-O-CH₂-COOH et de 75 % pour le [¹²⁵I]T₄-O-CH₂-COOH.

### II Etude de la liaison des dérivés de la sérotonine sur les récepteurs centraux

Le test a été réalisé avec le produit de l'exemple 1, stade B.

La distribution des sites de liaison du récepteur à haute affinité de la sérotonine (5-HT₁) est hétérogène dans le système nerveux central du rat (Pazos et Palacios, Brain Research, 346, 1985, p. 205-230, Ségu et al., Brain Research, 384, 1986, p. 205-217). De plus, le contenu en sous-types de sites 5-HT₁ varie selon la structure anatomique concernée.

Le protocole de préparation des coupes de cerveau est commun à toutes les études qui vont suivre. Les animaux sont décapités après anesthésie à l'hydrate de chloral (400 mg/kg poids brut). Les cerveaux, rapidement extraits de la boîte crânienne, sont congelés par immersion dans l'isopentane refroidi par l'azote liquide. Des coupes de 20 »m, réalisées au cryostat à -20°C, sont montées sur lames gélatinées et conservées à -20°C.

Les coupes sont pré-incubées 1 heure à 4°C dans une solution de Krebs (NaCl 118 mM ; KCl 4,8 mM ; CaCl₂ 1,2 mM, MgCl₂ 1,2 mM, Tris HCl 30 mM pH 7,4) pour éliminer les ligands endogènes.

### 1. Déplacement par les dérivés S-CM-R de la liaison à haute affinité de la sérotonine sur coupes de cerveau de rat.

### 1.1. Protocole d'incubation des coupes

Les incubations ont lieu pendant 60 minutes à 20°C dans une solution de Krebs contenant 10 »M de pargyline, 5,7 10⁻⁴ M d'acide ascorbique, 2 nM de [³H]5-HT (NEN, As = 30 Ci/mmole) et des concentrations croissantes de dérivés S-CM-R. La liaison non spécifique est déterminée sur des coupes homothétiques dans les mêmes conditions mais en présence de 5-HT 10⁻⁵ M.

Après incubation, les coupes sont rincées trois fois pendant 20 secondes dans l'eau distillée et séchées par un courant d'air chaud. Les coupes sont apposées à un film (Amersham) pendant six semaines, en présence d'étalon ([³H] microscale, Amersham). Les films sont développés 6 minutes avec du D19 Kodak, rincés et fixés avec AL4 (Kodak). L'analyse quantitative des radioautogrammes est menée avec un système d'analyse d'images (Ségu et al, J. Neurosci. Methods, 31, 1990, p. 197-208).

### 1.2. Résultats

La liaison spécifique de la [³H]5-HT est déplacée de façon monophasique par la 5-HT avec un IC₅₀ de 2 nM. Le S-O-CH₂-COOH (S-CM) déplace avec un IC₅₀ de 1000 nM, le S-CM-Gly-TyrNH₂ et le S-CM-Tyr-GlyNH₂ déplacent la liaison de façon biphasique avec des IC₅₀ de 20 nM pour le premier site et de 400 nM pour le deuxième.

### 2. Analyse des sites de liaison des dérivés iodés sur les récepteurs centraux du rat.

### 2.1. Protocole d'incubation des coupes.

Les incubations ont lieu pendant 60 minutes à 20°C dans une solution de Krebs contenant 10 »M de pargyline, 5,7 10⁻⁴ M d'acide ascorbique, 10 g/l de sérum albumine bovine (fraction V, sigma), et 0,03 nM du dérivé S-CM-[¹²⁵I]-R. La liaison non spécifique est déterminée sur des coupes homothétiques dans les mêmes conditions mais en présence de 5 HT 10⁻⁵ M.

Après incubation, les coupes sont rincées deux fois pendant 1 mn dans l'eau distillée et séchées par un courant d'air chaud. Les coupes sont apposées à un film (Amersham) pendant une semaine, en présence d'étalons ([¹²⁵I] microscale, Amersham). Les films sont développés 6 mn avec du D19 Kodak, rincés et fixés avec AL4 (Kodak). L'analyse quantitative des radioautogrammes est menée avec un système d'analyse d'images (Ségu et al., op. cité).

### 2.2. Distribution du marquage avec les fractions S-CM[¹²⁵I]R

### 2.2.1. Marquage par le S-CM[¹²⁵I]His

Les fractions de la préparation du dérivé S-CM [¹²⁵I]His (A.4.) ne marquent pas les coupes de cerveau de rat.

### 2.2.2. Marquage par le S-CM-G[¹²⁵I]TNH₂

Parmi les fractions recueillies lors de la préparation du dérivé iodé du S-CM-GTNH₂ (A.4.), seule la dernière fraction est retenue de façon spécifique sur les coupes de cerveau de rat. Cette fraction correspond au S-CM-G[¹²⁵I]TNH₂.

L'observation des radioautogrammes montre au niveau mesencéphalique un marquage intense de la substance noire (SN) et du subiculum dorsal (SD) et aucun marquage de l'hippocampe (H). Cette dernière structure est connue pour contenir presque exclusivement des sites de liaison de type 5-HT_{1A}, alors que les autres contiennent des sites de type 5-HT_{1B} (Hoyer et al, Eur. J. Pharmacol., 118, 1985, p. 1-12). Au niveau antérieur, le striatum (ST) est marqué : il contient surtout des sites 5-HT_{1B}, les plexus choroïdes (Px) contenant des sites 5-HT_{1C} ne le sont pas.

On peut en conclure que le S-CM-G[¹²⁵I]TNH₂ est un marqueur spécifique des sites de liaison à haute affinité de la sérotonine de type 1B.

### 3. Analyse de sites de liaison du S-CM-G[¹²⁵I]TNH₂ chez le cobaye

Dans le système nerveux central du cobaye, les structures anatomiques contenant des sites de type 5-HT_{1A} et la pharmacologie de ces sites sont les mêmes que chez le rat. Par contre, la pharmacologie des sites contenus dans la substance noire du cobaye est différente de celle des sites de la même structure chez le rat (Heuring and Peroutka, J. Neurosci., 7, 1987, p. 894-903). Aussi ces sites sont-ils désignés sous le vocable 5-HT_{1D}.

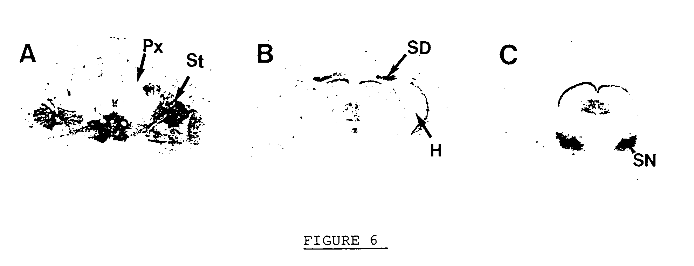

Avec la même méthode de traitement des coupes que celle utilisée en 2 pour le rat, on a démontré que chez le cobaye (figure 6), le S-CM-G[¹²⁵I]TNH₂ marque la substance noire ( figure 6C) et pas l'hippocampe ( figure 6B).

Le S-CM-G[¹²⁵I]TNH₂ est un marqueur des sites à haute affinité de la sérotonine de type 1D.

Ce dérivé de la 5-HT présente une affinité préférentielle pour les sites 5-HT₁B et 5-HT₁D par rapport aux sites 5-HT₁A et 5-HT₁C, ce qui fait de ce ligand un outil important d'analyse des récepteurs à haute affinité de la 5-HT.

Il est aujourd'hui le seul à présenter une sélectivité aussi importante, sans se lier à d'autres types de récepteurs non 5-HT (comme les β-adrénergiques). Par ailleurs, il est le seul à marquer les 5-HT₁D. Il peut donc être utilisé pour les études de récepteurs de ce type chez l'homme et leur variation dans les maladies neurodégénératives (chorée de Huntington).

### 4. Analyse des sites de liaisons chez le singe.

Le test a été réalisé avec le dérivé de l'exemple 2, stade A.

### A. Préparation biologique :

Un singe mâle (Macacca mulatta n.) de 8 kg a été sacrifié avec une surdose de barbituriques et exsanguination. Une fois la mort de l'animal constatée, la boîte crânienne est enlevée et le cerveau mis à nu. L'hémicerveau droit est découpé et congelé par l'isopentane refroidi dans l'azote liquide. Le bloc est conservé à - 20° C. Des coupes de 10 »m d'épaisseur sont réalisées au cryostat à -20° C, et posées sur des lames gélatinées (Ségu. et al. 1990, J. Neurosci. meth. 31, 197). Elles sont conservées à -20° C jusqu'à utilisation.

### - Incubation avec la sonde radioactive :

Les coupes sont préincubées 1h à 4° C dans une solution de Krebs (mM : NaCl, 118 ; KCl 4,5 ; CaCl₂, 1,2; MgCl₂, 1,2 ; Tris, 15 ; pH 7,4) afin d'éliminer les ligands endogènes. Elles sont ensuite incubées à 20° C pendant 60 minutes dans une solution contenant de la pargyline 10⁻⁵M, de l'acide ascorbique 57 mM, 10 g/l de sérum albumine bovine et du S-CM-G[¹²⁵I] TNH₂ 0,02 nM seul ou en présence de concentrations croissantes de sérotonine. Certaines coupes sont incubées dans des conditions identiques, mais la sonde radioactive est remplacée soit par [³H]5-HT 2 nM, soit par [³H]5-HT 2 nM en présence de 100 nM de 8-hydroxy-2-[di-N-propylamino] tétraline (8-OH-DPAT) et 100 nM de mésulergine, soit par [³H]8-OH-DPAT 1 nM. Après rinçage 2 x 1 minute dans l'eau distillée, elles sont séchées.

### - Radioautographie :

Les lames sont apposées sur un film sensible au tritium pendant 8 jours. Le film est développé 6 minutes dans le D19, rinçé et fixé. Les autoradiogrammes sont quantifiés avec un système vidéo d'analyse d'images (Ségu. et al, 1990).

### B. Résultats

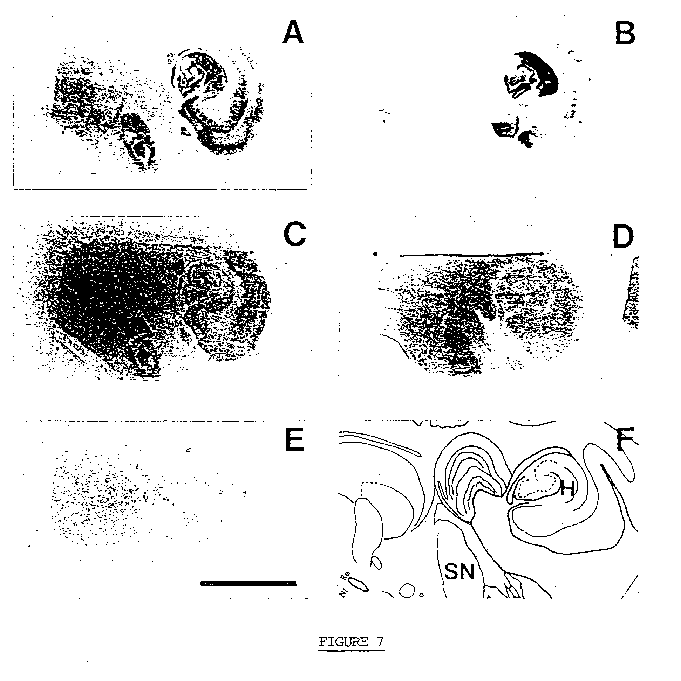

L'incubation en présence de [³H]5-HT 2 nM (Fig. 7A) montre un marquage important de l'hippocampe et de la substance noire. Dans ces conditions, tous les types de sites 5-HT_{1A} sont marqués.

L'incubation en présence de [³H]8-OH-DPAT 1 nM (Fig. 7B) qui ne marque que les sites 5-HT_{1A}, montre un marquage intense de l'hippocampe et pas de la substance noire.

Dans le cas de l'incubation dans [³H]5-HT en présence de 100 nM 8-OH-DPAT et mésulergine (Fig. 7C), seuls les sites 5-HT_{1D} sont marqués. Les autoradiogrammes montrent une forte réaction sur la substance noire.

L'incubation dans le S-CM-G[¹²⁵I]TNH₂ 0,02 nM seul (Fig. 7D) montre un marquage quasi-exclusif de la substance noire, comme dans le cas précédent. Ce dérivé O-carboxyméthylé de la sérotonine se lie aux sites 5-HT_{1D}.

Si on déplace la liaison du S-CM-G[¹²⁵I]TNH₂ 0,02 nM par la sérotonine, on obtient une IC₅₀ (concentration inhibant 50 % de la liaison) de l'ordre de 5 nM, montrant donc que la liaison du dérivé est spécifique des sites 5-HT_{1D}.

### Conclusion :

Le S-CM-G[¹²⁵I]TNH₂ est un marqueur des récepteurs 5-HT_{1D}. Toute molécule analogue aura donc cette propriété de se lier aux sites 5-HT_{1D}.

### 5. Analyse des sites de liaisons de S-CM-G[¹²⁵I]TNH₂ chez l'homme

a) Les cerveaux humains post-mortem sont conservés à -20° C. Des coupes de 10 »m sont réalisées sur des blocs contenant soit le locus niger, soit la corne de Amon. Les coupes sont traitées comme indiqué en 4 pour les coupes de cerveau de singe.
b) Après radioautographie, on observe un fort marquage du locus niger (structure correspondant à la substance noire chez le rat) et un marquage faible sur le corne de Amon (équivalent chez l'homme de l'hippocampe du rat).

Le locus niger contient quasi exclusivement des sites de liaisons de type 5-HT_{1D}, alors que la corne de Amon est composée en majorité de sites de type 5-HT_{1A}.

Le dérivé iodé testé marque donc in vitro les récepteurs 5-HT_{1D} du cerveau humain. Il peut donc être utilisé pour les études de récepteurs de ce type chez l'homme et leur variation dans les maladies neurodégénératives (chorée de Huntington). Les dérivés décrits dans le présent brevet qui présentent cette propriété et qui passent la barrière hémato-encéphalique, pourront être utilisés à des fins thérapeutiques dans le cas de dysfonctionnements liés au type de récepteurs concernés (5-HT_{1D}).

### III Liaison des dérivés de la sérotonine sur les récepteurs périphériques

### 1. Franchissement de la barrière hémato-encéphalique.

Lorsque les molécules passent la barrière hémato-encéphalique, elles sont suceptibles d'agir sur les récepteurs du système nerveux central, effet recherché dans de nombreux cas (cf. point 4).

Dans le cas où les récepteurs existent aussi bien en périphérie que dans le système nerveux central, il peut être souhaitable d'avoir des dérivés qui ne passent pas la barrière hémato-encéphalique. Ces dérivés auront alors un effet sur les récepteurs périphériques, sans action centrale prédominante.

### A. Protocole expérimental

2 souris et 2 cobayes sont anesthésiés profondément à l'hydrate de chloral (injection intrapéritonéale de 0,12 ml pour 100 g de poids d'une solution à 35 %). La cage thoracique est ouverte, le coeur est mis à nu.

Une injection intracardiaque de S-CM-G[¹²⁵I]TNH₂ de 200 »l pour la souris et 1,5 ml pour le cobaye est réalisée.

Après 10 minutes, les animaux, toujours sous anesthésie profonde sont décapités, les cerveaux congelés et conservés comme indiqué dans l'exemple 1.

On procède à des coupes et à une autoradiographie comme décrit dans l'exemple 1.

### B. Résultats

L'examen de coupes passant en divers niveaux du cerveau ne montre aucun marquage, en particulier dans la substance noire, structure anatomique qui est bien marquée lorsque l'on procède à une incubation in vitro dans le même traceur.

### Conclusion :

Le dérivé de la sérotonine testé, ne passe pas la barrière hémato-encéphalique, permettant de soulager les effets les plus douloureux de la migraine. Aucune toxicité n'a été observée à la dose injectée.

### 2. Etude par imagerie in vivo avec une gamma caméra de la répartition de la liaison du dérivé S-CM-G[¹²⁵I]TNH₂

Etant donné que ce dérivé ne passe pas la barrière hématoencéphalique comme nous l'avons montré en 1, nous avons étudié la distribution du marquage périphérique par imagerie in vivo.

Les animaux (souris) sont anesthésiés et injectés par voie intraveineuse avec du dérivé S-CM-G[¹²⁵I]TNH₂. Très rapidement, on observe la diffusion du produit dans le coeur, tout le système vasculaire et le foie. 10 minutes après l'injection, le marquage diminue dans le système vasculaire, sauf le coeur et le tronc vasculaire cérébral. Dans cette dernière zone, le marquage est retenu pendant 15 à 20 minutes, avant de disparaître lentement. Le marquage de la vessie s'accroît à des temps plus longs, le coeur restant marqué assez longtemps.

Une expérience a été réalisée avec un dérivé iodé, présentant la même liaison spécifique que le dérivé ci-dessus, mais ne possédant pas la propriété de se lier spécifiquement sur les sites 5-HT_{1B} et 5-HT_{1D} centraux. Dans ce cas, le décours du marquage observé avec la gamma caméra est identique à celui observé avec le S-CM-G[¹²⁵I]-TNH₂, sauf pour le tronc vasculaire cérébral, qui ne présente pas de rétention particulière.

Le S-CM-GTNH₂ et ses dérivés, peuvent donc être utilisés à des fins thérapeutiques dans le soulagement des crises de migraine. Ne pouvant franchir la barrière hématoencéphalique, ils ne sont pas susceptibles de provoquer des effets secondaires par action sur les sites sérotoninergiques centraux de l'homme.

### EXEMPLE 3

On a préparé des comprimés répondant à la formule :

| | |
|---|---|
| 5-0-carboxyméthylglycyltyrosinamide (S-CM-GTNH₂) | 0,5 mg |
| excipient q.s. pour un comprimé terminé à | 100 mg |
| (détail de l'excipient : lactose, amidon, talc, stéarate de magnésium) | |

### EXEMPLE 4

On a préparé des comprimés sécables répondant à la formule :

| | |
|---|---|
| 5-0-carboxyméthylglycyltyrosinamide (S-CM-GTNH₂) | 2 mg |
| excipient q.s. pour un comprimé terminé à | 100 mg |
| (détail de l'excipient : lactose, amidon, talc, stéarate de magnésium). | |

### EXEMPLE 5

On a préparé une préparation injectable répondant à la formule :

| | |
|---|---|
| 5-0-carboxyméthylglycyltyrosinamide (S-CM-GTNH₂) | 2 mg |
| excipient : eau pour préparation injectable | 2 ml |

### EXEMPLE 6

On a préparé un aérosol nasal répondant à la formule :

| | |
|---|---|
| 5-0-carboxyméthylglycyltyrosinamide (S-CM-GTNH₂) | 30 mg |
| excipient : soluté aqueux : chlorure de sodium, citrate trisodique, acide citrique, | |
| eau distillée | 15 ml |

### EXEMPLE 7

On a préparé un aérosol buccal répondant à la formule :

| | |
|---|---|
| 5-0-carboxyméthylglycyltyrosinamide (S-CM-GTNH₂) | 60 mg |
| excipient : soluté aqueux : acide citrique, alcool éthylique, édulcorant, aromatisant, polysorbate 80, propylèneglycol, | |
| eau purifiée | 50 ml |
| Propulseur : azote | |

### EXEMPLE 8 : Kit d'analyse d'un récepteur 5-HT_{1D}

On a préparé un kit d'étude de présence du récepteur 5-HT_{1D}, d'affinité de dérivé pour ledit récepteur et de révélation des altérations de l'affinité et du nombre de ces récepteurs, ayant la composition suivante :
- Produit de l'exemple 2, stade B (2000 Ci/mmole) dans du

| | |
|---|---|
| tampon Tris HCL 50 mM pH 7,4 | 15 ml |

- Standard sérotonine (oxalate) lyophilisée, 10 nmoles, et tampon pH 6,2
- Standard produit de l'exemple 2, stade B, 1 nmole de sérotonine par flacon, et tampon pH 6,2
- Solution d'inhibiteur : pargyline 1,25 mmole, 8-OH-DPAT 12,5 mmoles, mésulergine 12,5 nmoles par flacon, tampon pH 7,4
- Diluant : tampon salin pH 7,4
- Filtres : fibre de verre

### LEGENDE DES FIGURES

**Figure 1 :** Purification des produits de la carboxyméthylation de la N-BOC-T₃
   A : chromatogramme après CLHP » Bondapack C₁₈ 0-30 mn : TFA 0,05 % 50 vol, méthanol 50 vol 30-80 mn : gradient méthanol 100 %.
      La hauteur des pics n'est pas absolue car il s'agit de deux profils d'élution différents superposés.
   B : spectre d'absorption du BOC-T₃ en milieu acide (Ac) et alcalin (Al). Ordonnées : D.O.
      Abscisses : longueur d'ondes de 220 à 350 nm. Le spectre est déplaçable en milieu basique, comme pour la T₃.
   C : spectre d'absorption du BOC-T₃-OCH₂-COOH en milieu acide (Ac) et alcalin (Al).
      Ordonnées : D.O. - Abscisses : longueur d'ondes de 220 à 350 nm. Le spectre n'est pas déplaçable en milieu basique.

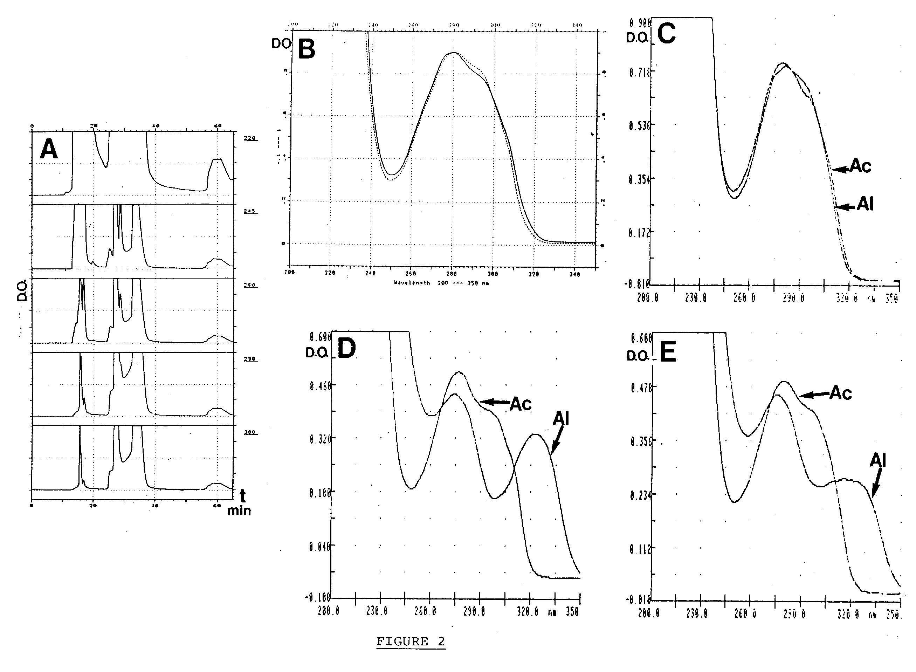

**Figure 2 :** Purification des produits de la carboxyméthylation de la sérotonine-N-BOC (BOC-S)
   A : chromatogramme après CLHP sur Ultrasphère ODS C₁₈ en isocratique H₂O-TFA 0,05 % 55 vol, méthanol 45 vol.
      En ordonnées : densité optique (D.O.) pour diverses longueurs d'ondes (indiquées à droite). En abscisses, le temps en minutes.
   B : spectre d'absorption ajusté à 280 nm du produit sortant à 28 minutes, le BOC-S (trait plein) et de celui sortant à 36 minutes, le BOC-S-CM (trait pointillé). Ordonnées : D.O., abscisses : longueurs d'ondes 200 à 350 nm.
   C : spectre d'absorption du BOC-S-CM en milieu acide (Ac) et alcalin (Al). Ordonnées : D.O.-Abscisses : longueur d'ondes 200 à 350 nm.
   D : spectre d'absorption de la sérotonine en milieu acide (Ac) et alcalin (Al). Ordonnées : D.O. - Abscisses : longueur d'ondes 200 à 350 nm.
   E : spectre d'absorption du BOC-S- en milieu acide (Ac) et alcalin (Al). Ordonnées : D.O.-Abscisses : longueur d'ondes 200 à 350 nm.
      Remarquer que le spectre est déplacé en milieu basique comme pour la sérotonine (figure 1D). Ce n'est pas le cas du BOC-S-CM (figure 1C) qui est substitué sur le OH.

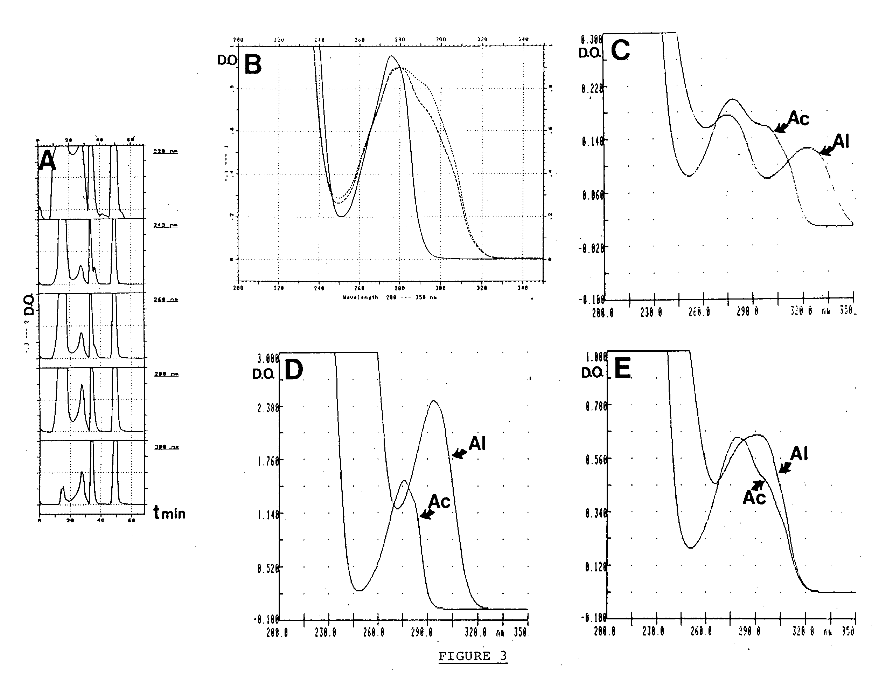

**Figure 3 :** Purification des produits de la conjugaison du BOC-S-CM et du GTNH₂
   A : chromatogramme après CLHP sur Ultrasphère C₁₈ en isocratique H₂0 pH 6 60 vol, méthanol 40 vol.
      Ordonnées : D.O. pour diverses longueurs d'ondes (indiquées à droite). En abscisses, le temps en minutes.
   B : spectre d'absorption ajusté à 280 nm du produit sortant à 18 minutes, le GTNH₂ (trait plein) de celui sortant à 35 minutes, le BOC-S-CM (trait pointillé court) et de celui sortant à 48 minutes, le BOC-S-CM-GTNH₂ (trait pointillé long). Ordonnées : D.O. - Abscisses : longueur d'ondes de 200 à 350 nm.
   C : spectre d'absorption du BOC-S en milieu acide (Ac) et alcalin (Al). Ordonnées : D.O.-Abscisses : longueur d'ondes de 200 à 350 nm.
   D : spectre d'absorption du GTNH₂ en milieu acide (Ac) et alcalin (Al). Ordonnées : D.O.-Abscisses : longueur d'ondes de 200 à 350 nm.
   E : spectre d'absorption du BOC-S-CM-GTNH₂ en milieu acide (Ac) et alcalin (Al). Ordonnées : D.O. - Abscisses : longueur d'ondes de 200 à 350 nm. Remarquer que le déplacement du spectre en milieu alcalin ne correspond pas du tout à celui d'une sérotonine non substituée sur le OH (figure 2C), tout en étant plus déplacé que le spectre du BOC-S-CM (figure 1C). On observe un déplacement vers les grandes longueurs d'ondes, comme pour le GTNH₂ (figure 2D), mais cette modification est moins élevée en D.O.

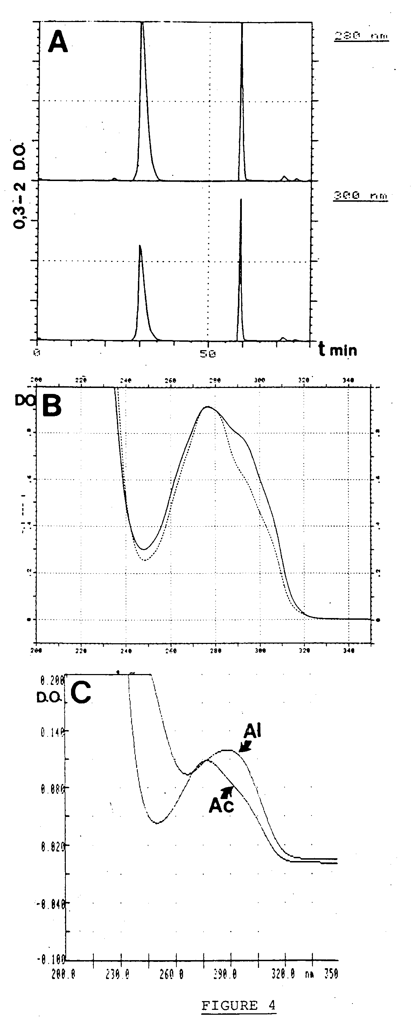

**Figure 4 :** Purification des produits de déprotection de l'amine du BOC-S-CM-GTNH₂
   A : chromatogramme après CLHP sur C₁₈ Bondapack en isocratique H₂0-TFA 0,05 % 65 vol, méthanol 35 vol.
      Ordonnées : D.O. pour 280 et 300 nm - Abscisses, temps en minutes.
   B : spectre d'absorption ajusté à 280 nm du produit sortant à 24 minutes, le S-CM (trait plein) et celui sortant à 30 minutes, le S-CM-GTNH₂ (trait pointillé). Le spectre du produit sortant à 60 minutes, le BOC-S-CM-GTNH₂ est identique à celui montré sur la figure 2B.
   C : spectre d'absorption du S-CM-GTNH₂ en milieu acide (Ac) et alcalin (Al). Ordonnées : D.O. - Abscisses : longueur d'ondes de 200 à 350 nm. Remarquer que le déplacement du spectre en fonction du pH est identique à celui obtenu pour le BOC-S-CM-GTNH₂ montré sur la figure 2E.

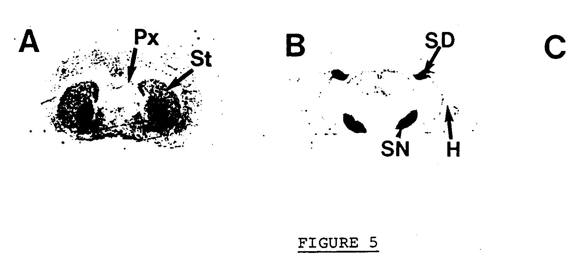

**Figure 5 :** Analyse des sites de liaison du S-CM-G[¹²⁵I]-TNH₂ chez le rat - Coupes (10 »m) de cerveau de rat incubées dans S-CM-G[¹²⁵I]TNH₂ 0,03 nM.
   A : au niveau antérieur
      Px : plexus choroïdes ; St : striatum
   B : au niveau mésencéphalique
      SD : subiculum dorsal ; SN : substance noire ;
      H : hippocampe
   C : liaison non spécifique au niveau mésencéphalique établie en présence de 5-HT 10⁻⁵ M Remarquer que Px et H ne sont pas marqués (alors qu'ils contiennent respectivement des sites 5-HT_{1C} et 5-HT_{1A}) et que St, SD et SN (qui possèdent des sites 5-HT_{1B}) sont marqués.
**Figure 6 :** Analyse des sites de liaison du S-CM-G[¹²⁵I]-TNH₂ chez le cobaye - Coupes (10 »m) de cerveau de cobaye incubées dans S-CM-G[¹²⁵I]TNH₂ 0,03 nM.
   A : au niveau antérieur
      Px : plexus choroïdes ; St : striatum
   B : au niveau hippocampe
      SD : subiculum dorsal ; H : hippocampe
   C : Niveau substance noire :
      SN : substance noire
      Remarquer que Px et H ne sont pas marqués (alors qu'ils contiennent respectivement des sites 5-HT_{1C} et 5-HT_{1A}) et que St, SD et SN (qui possèdent des sites 5-HT_{1D}) sont marqués.
**Figure 7** : Détection des récepteurs 5-HT₁ sur coupes de cerveau de singe (Macacca mulatta n). Les coupes (10 »m) de tissu congelé sont incubés avec diverses sondes et radioautographiées.
   A. Incubation dans ^{[}3H]5-HT 2 nM montrant les sites 5-HT₁ totaux dans l'hippocampe (H), la substance noire (SN).
   B. Incubation dans ^{[}3H]8-OH-DPAT 1 nM montrant les sites 5-HT₁ₐ exclusivement localisés dans l'hippocampe.
   C. Incubation dans [³H]5-HT 2 nM de 100 nM 8-OH-DPAT et 100 nM mésulergine, montrant les sites 5-HT_{1B} quasi exclusivement localisés dans la substance noire.
   D. Incubation [¹²⁵I]S-CM-GTNH₂ 0,02 nM, montrant un marquage exclusif de la substance noire, similaire à celui montré en C.
   E. Même incubation que D mais additionné de 10⁻⁵ M 5HT montrant la liaison non spécifique (échelle = 1 cm).
   F. Schéma des structures anatomiques, tiré au niveau A11,5 de l'atlas Riche et al. 1968.

## Revendications (Revendications pour l'(les) Etat(s) contractant(s) suivant(s): AT, BE, CH, DE, DK, FR, GB, IT, LI, LU, NL, SE)

1. Les dérivés de molécules biologiquement actives comportant une fonction amine primaire et un noyau hydroxylé ainsi que leurs sels d'addition avec les acides minéraux ou organiques, caractérisés en ce qu'ils répondent à la formule générale I :
[R'R''N-A-B-O-CH₂-CO]ₙR₁ (I)
dans laquelle
n représente un nombre entier de 1 à 10 ;
A représente une chaîne alcoylène linéaire ou ramifiée renfermant de 1 à 5 atomes de carbone ;
B représente un noyau aromatique comportant de 6 à 10 atomes de carbone, le cas échéant substitués et comportant éventuellement un hétéroatome,
- R₁ représente un reste aminé, un reste alcool choisi parmi les phénols éventuellement substitués et les alcools aliphatiques en C₁-C₁₆,
et
R' et R'' représentent un radical alcoyle renfermant de 1 à 5 atomes de carbone ou un atome d'hydrogène, ainsi que leurs sels d'addition avec les acides minéraux ou organiques.

2. Les dérivés de formule (I) tels que définis à la revendication 1, ainsi que leurs sels d'addition avec les acides minéraux ou organiques, caractérisés en ce que B représente un noyau phényle ou indole, éventuellement substitué par un ou plusieurs atomes d'halogène.

3. Les dérivés selon la revendication 2, ainsi que leurs sels d'addition avec les acides minéraux ou organiques, caractérisés en ce que R₁ représente un radical NHR dans lequel R représente un reste d'une diamine, d'une protéine, d'un acide aminé ou d'un polypeptide constitué au plus de 5 acides aminés ou des dérivés desdits acides aminés ou polypeptides.

4. Les dérivés selon la revendication 1, 2 ou 3, caractérisés en ce que R' et R'' représentent un radical alkyle linéaire ou ramifié renfermant de 1 à 5 atomes de carbone, et A représente un radical -CH₂-CH₂-, ainsi que leurs sels d'addition avec les acides minéraux ou organiques.

5. L'un des dérivés dont les noms suivent :
- le tryptamine-5-O-carboxyméthylglycyltyrosinamide [S-CM-GTNH₂],
- le tryptamine-5-O-carboxyméthyltyrosylglycinamide
- ainsi que leurs sels d'addition avec les acides minéraux ou organiques.

6. L'un des dérivés dont les noms suivent :
- le tryptamine-5-O-carboxyméthylglycyliodotyrosinamide [S-CM-iodoGTNH₂],
- le tryptamine-5-O-carboxyméthyliodotyrosylglycinamide
- ainsi que leurs sels d'addition avec les acides minéraux ou organiques

7. Les dérivés de molécules biologiquement actives comportant une fonction amine primaire et un noyau hydroxylé ainsi que leurs sels d'addition avec les acides minéraux ou organiques, caractérisés en ce qu'ils répondent à la formule :
R'R''N-A-B-O-CH₂-CO-NH-R-NH-CO-CH₂-O-B-A-N R' R''
dans laquelle A, B, R' et R'' ont la signification indiquée à la revendication 1, et R celle indiquée à la revendication 10, ou leurs amides.

8. Les dérivés selon l'une quelconque des revendications 1 à 5, caractérisés en ce qu'ils se présentent sous une forme marquée, le marqueur pouvant être un élément radioactif, en particulier ¹²⁵I, une molécule fluorescente, une enzyme, une protéine portant un or colloïdal ou un produit de contraste.

9. Les dérivés selon l'une quelconque des revendications 1 à 5, caractérisés en ce qu'ils comportent une protéine greffée sur le chaînon O-carboxyméthyle selon le procédé de la revendication 10.

10. Procédé de préparation des dérivés de formule (I) tels que définis à la revendication 1, ainsi que de leurs sels d'addition avec les acides minéraux ou organiques, caractérisé en ce que l'on fait réagir un dérivé de formule (II) :
R'R'' N-A-B-OH (II)
dans laquelle R', R'', A et B ont la signification déjà indiquée, avec un dérivé comportant un groupement protecteur des fonctions amine dans le cas où R' = R''= H, pour obtenir un dérivé de formule (III) :
Y-HN-A-B-OH (III)
dans laquelle A et B ont la signification déjà indiquée et Y représente un groupement protecteur des fonctions amine, facilement clivable, que l'on fait réagir avec un acide halogéno-acétique pour obtenir un dérivé de formule (IV) :
Y-HN-A-B-O-CH₂COOH (IV)
dans laquelle A, B et Y ont la signification déjà indiquée, que l'on fait réagir avec un dérivé aminé ou un dérivé d'un alcool pour obtenir le dérivé de formule (I) telle que définie ci-dessus, le cas échéant sous forme dimère de formule (I') :
R'R''-N-A-B-O-CH₂-CO-NH-R-NH-CO-CH₂-O-B-A-N-R'R'' (I')
dans le cas d'une diamine ou polymère dans le cas d'une polyamine, R représentant le reste correspondant de diamine ou de polyamine, que l'on isole et, si désiré, salifie.

11. Compositions pharmaceutiques caractérisées en ce qu'elles renferment à titre de principe actif l'un au moins des médicaments tels que définis à l'une des revendications 1 à 9.

12. Application des dérivés selon l'une quelconque des revendications 4 et 5 à l'imagerie des sites de liaison des médiateurs endogènes.

13. Application des dérivés selon l'une quelconque des revendications 5 à 8 à la purification des récepteurs des médiateurs endogènes.

14. Application des dérivés selon la revendication 9, à la préparation d'anticorps dirigés contre les médiateurs endogènes et leurs dérivés.

15. Kits d'analyse pour le dosage des médiateurs et de leurs dérivés, caractérisés en ce qu'ils renferment l'un au moins des dérivés selon l'une quelconque des revendications 1 à 9.

## Revendications (Revendications pour l'(les) Etat(s) contractant(s) suivant(s): ES)

1. Procédé de préparation, dans lequel l'on prépare un dérivé de molécules biologiquement actives comportant une fonction amine primaire et un noyau hydroxylé ou l'un de ses sels d'addition avec les acides minéraux ou organiques, caractérisé en ce qu'il répond à la formule générale I :
[R'R''N-A-B-O-CH₂-CO]ₙR₁ (I)
dans laquelle
n représente un nombre entier de 1 à 10 ;
A représente une chaîne alcoylène linéaire ou ramifiée renfermant de 1 à 5 atomes de carbone ;
B représente un noyau aromatique comportant de 6 à 10 atomes de carbone, le cas échéant substitués et comportant éventuellement un hétéroatome,
- R₁ représente un reste aminé, un reste alcool choisi parmi les phénols éventuellement substitués et les alcools aliphatiques en C₁-C₁₆,
et
R' et R'' représentent un radical alcoyle renfermant de 1 à 5 atomes de carbone ou un atome d'hydrogène, ainsi que leurs sels d'addition avec les acides minéraux ou organiques, caractérisé en ce que l'on fait réagir un dérivé de formule (II) :
R'R'' N-A-B-OH (II)
dans laquelle R', R'', A et B ont la signification déjà indiquée, avec un dérivé comportant un groupement protecteur des fonctions amine dans le cas où R' = R''= H, pour obtenir un dérivé de formule (III) :
Y-HN-A-B-OH (III)
dans laquelle A et B ont la signification déjà indiquée et Y représente un groupement protecteur des fonctions amine, facilement clivable, que l'on fait réagir avec un acide halogéno-acétique pour obtenir un dérivé de formule (IV) :
Y-HN-A-B-O-CH₂COOH (IV)
dans laquelle A, B et Y ont la signification déjà indiquée, que l'on fait réagir avec un dérivé aminé ou un dérivé d'un alcool pour obtenir le dérivé de formule (I) telle que définie ci-dessus, le cas échéant sous forme dimère de formule (I') :
R'R''-N-A-B-O-CH₂CO-NH-R-NH-CO-CH₂-O-B-A-N-R'R'' (I')
dans le cas d'une diamine ou polymère dans le cas d'une polyamine, R représentant le reste correspondant de diamine ou de polyamine, que l'on isole et, si désiré, salifie.

2. Procédé selon la revendication 1, caractérisé en ce que l'on prépare un dérivé de formule (I) tel que défini à la revendication 1, ou l'un de ses sels d'addition avec les acides minéraux ou organiques, dans lequel B représente un noyau phényle ou indole, éventuellement substitué par un ou plusieurs atomes d'halogène.

3. Procédé selon la revendication 2, caractérisé en ce que l'on prépare un dérivé ou l'un de ses sels d'addition avec les acides minéraux ou organiques, dans lequel R₁ représente un radical NHR dans lequel R représente un reste d'une diamine, d'une protéine, d'un acide aminé ou d'un polypeptide constitué au plus de 5 acides aminés ou des dérivés desdits acides aminés ou polypeptides.

4. Procédé selon la revendication 1, 2 ou 3, caractérisé en ce que l'on prépare un dérivé ou l'un de ses sels d'addition avec les acides minéraux ou organiques, dans lequel R' et R'' représentent un radical alkyle linéaire ou ramifié renfermant de 1 à 5 atomes de carbone, et A représente un radical -CH₂-CH₂-, ou l'un de ses sels d'addition avec les acides minéraux ou organiques.

5. Procédé de préparation selon la revendication 1 caractérisé en ce que l'on prépare l'un des dérivés dont les noms suivent :
- le tryptamine-5-O-carboxyméthylglycyltyrosinamide [S-CM-GTNH₂],
- le tryptamine-5-O-carboxyméthyltyrosylglycinamide
- ou l'un de leurs sels d'addition avec les acides minéraux ou organiques.

6. Procédé de préparation selon la revendication 1, caractérisé en ce que l'on prépare l'un des dérivés dont les noms suivent :
- le tryptamine-5-O-carboxyméthylglycyliodotyrosinamide [S-CM-iodoGTNH₂].
- le tryptamine-5-O-carboxyméthyliodotyrosylglycinamide
- ou l'un de leurs sels d'addition avec les acides minéraux ou organiques

7. Procédé de préparation selon la revendication 1, caractérisé en ce que l'on prépare un dérivé de molécules biologiquement actives comportant une fonction amine primaire et un noyau hydroxylé ou l'un de ses sels d'addition avec les acides minéraux ou organiques, répondant à la formule :
R'R''N-A-B-O-CH₂-CO-NH-R-NH-CO-CH₂-O-B-A-N R'R''
dans laquelle A, B, R' et R'' ont la signification indiquée à la revendication 1, et R celle indiquée à la revendication 10, ou un de ses amides.

8. Procédé de préparation, caractérisé en ce que l'on prépare un dérivé selon l'une quelconque des revendications 1 à 5, se présentant sous une forme marquée, le marqueur pouvant être un élément radioactif, en particulier ¹²⁵I, une molécule fluorescente, une enzyme, une protéine portant un or colloïdal ou un produit de contraste.

9. Procédé de préparation, caractérisé en ce que l'on prépare un dérivé selon l'une quelconque des revendications 1 à 5, comportant une protéine greffée sur le chaînon O-carboxyméthyle selon le procédé de la revendication 1.

10. Procédé de préparation de compositions pharmaceutiques caractérisé en ce que lesdites compositions renferment à titre de principe actif l'un au moins des médicaments tels que définis à l'une des revendications 1 à 9 dans lequel on mélange ledit principe actif avec un excipient pharmaceutiquement acceptable.

11. Application des dérivés selon l'une quelconque des revendications 4 et 5 à l'imagerie des sites de liaison des médiateurs endogènes.

12. Application des dérivés selon l'une quelconque des revendications 5 à 8 à la purification des récepteurs des médiateurs endogènes.

13. Application des dérivés selon la revendication 9, à la préparation d'anticorps dirigés contre les médiateurs endogènes et leurs dérivés.

14. Kits d'analyse pour le dosage des médiateurs et de leurs dérivés, caractérisés en ce qu'ils renferment l'un au moins des dérivés selon l'une quelconque des revendications 1 à 9.

## Revendications (Revendications pour l'(les) Etat(s) contractant(s) suivant(s): GR)

1. Les dérivés de molécules biologiquement actives comportant une fonction amine primaire et un noyau hydroxylé ainsi que leurs sels d'addition avec les acides minéraux ou organiques, caractérisés en ce qu'ils répondent à la formule générale I :
[R'R''N-A-B-O-CH₂-CO]ₙR₁ (I)
dans laquelle
n représente un nombre entier de 1 à 10 ;
A représente une chaîne alcoylène linéaire ou ramifiée renfermant de 1 à 5 atomes de carbone;
B représente un noyau aromatique comportant de 6 à 10 atomes de carbone, le cas échéant substitués et comportant éventuellement un hétéroatome,
- R₁ représente un reste aminé, un reste alcool choisi parmi les phénols éventuellement substitués et les alcools aliphatiques en C₁-C₁₆,
et
R' et R'' représentent un radical alcoyle renfermant de 1 à 5 atomes de carbone ou un atome d'hydrogène, ainsi que leurs sels d'addition avec les acides minéraux ou organiques.

2. Les dérivés de formule (I) tels que définis à la revendication 1, ainsi que leurs sels d'addition avec les acides minéraux ou organiques, caractérisés en ce que B représente un noyau phényle ou indole, éventuellement substitué par un ou plusieurs atomes d'halogène.

3. Les dérivés selon la revendication 2, ainsi que leurs sels d'addition avec les acides minéraux ou organiques, caractérisés en ce que R₁ représente un radical NHR dans lequel R représente un reste d'une diamine, d'une protéine, d'un acide aminé ou d'un polypeptide constitué au plus de 5 acides aminés ou des dérivés desdits acides aminés ou polypeptides.

4. Les dérivés selon la revendication 1, 2 ou 3, caractérisés en ce que R' et R'' représentent un radical alkyle linéaire ou ramifié renfermant de 1 à 5 atomes de carbone, et A représente un radical -CH₂-CH₂-, ainsi que leurs sels d'addition avec les acides minéraux ou organiques.

5. L'un des dérivés dont les noms suivent :
- le tryptamine-5-O-carboxyméthylglycyltyrosinamide [S-CM-GTNH₂],
- le tryptamine-5-O-carboxyméthyltyrosylglycinamide
- ainsi que leurs sels d'addition avec les acides minéraux ou organiques.

6. L'un des dérivés dont les noms suivent :
- le tryptamine-5-O-carboxyméthylglycyliodotyrosinamide [S-CM-iodoGTNH₂],
- le tryptamine-5-O-carboxyméthyliodotyrosylglycinamide
- ainsi que leurs sels d'addition avec les acides minéraux ou organiques

7. Les dérivés de molécules biologiquement actives comportant une fonction amine primaire et un noyau hydroxylé ainsi que leurs sels d'addition avec les acides minéraux ou organiques, caractérisés en ce qu'ils répondent à la formule :
R'R''N-A-B-O-CH₂-CO-NH-R-NH-CO-CH₂-O-B-A-N R'R''
dans laquelle A, B, R' et R'' ont la signification indiquée à la revendication 1, et R celle indiquée à la revendication 10, ou leurs amides.

8. Les dérivés selon l'une quelconque des revendications 1 à 5, caractérisés en ce qu'ils se présentent sous une forme marquée, le marqueur pouvant être un élément radioactif, en particulier ¹²⁵I, une molécule fluorescente, une enzyme, une protéine portant un or colloïdal ou un produit de contraste.

9. Les dérivés selon l'une quelconque des revendications 1 à 5, caractérisés en ce qu'ils comportent une protéine greffée sur le chaînon O-carboxyméthyle selon le procédé de la revendication 10.

10. Procédé de préparation des dérivés de formule (I) tels que définis à la revendication 1, ainsi que de leurs sels d'addition avec les acides minéraux ou organiques, caractérisé en ce que l'on fait réagir un dérivé de formule (II) :
R'R'' N-A-B-OH (II)
dans laquelle R', R'', A et B ont la signification déjà indiquée, avec un dérivé comportant un groupement protecteur des fonctions amine dans le cas où R' = R''= H, pour obtenir un dérivé de formule (III) :
Y-HN-A-B-OH (III)
dans laquelle A et B ont la signification déjà indiquée et Y représente un groupement protecteur des fonctions amine, facilement clivable, que l'on fait réagir avec un acide halogéno-acétique pour obtenir un dérivé de formule (IV) :
Y-HN-A-B-O-CH₂COOH (IV)
dans laquelle A, B et Y ont la signification déjà indiquée, que l'on fait réagir avec un dérivé aminé ou un dérivé d'un alcool pour obtenir le dérivé de formule (I) telle que définie ci-dessus, le cas échéant sous forme dimère de formule (I') :
R'R''-N-A-B-O-CH₂-CO-NH-R-NH-CO-CH₂-O-B-A-N-R'R'' (I')
dans le cas d'une diamine ou polymère dans Le cas d'une polyamine, R représentant le reste correspondant de diamine ou de polyamine, que l'on isole et, si désiré, salifie.

11. Procédé de préparation de compositions pharmaceutiques caractérisées en ce qu'elles renferment à titre de principe actif l'un au moins des médicaments tels que définis à l'une des revendications 1 à 9 dans lequel on mélange ledit principe actif avec un excipient pharmaceutiquement acceptable.

12. Application des dérivés selon l'une quelconque des revendications 4 et 5 à l'imagerie des sites de liaison des médiateurs endogènes.

13. Application des dérivés selon l'une quelconque des revendications 5 à 8 à la purification des récepteurs des médiateurs endogènes.

14. Application des dérivés selon la revendication 9, à la préparation d'anticorps dirigés contre les médiateurs endogènes et leurs dérivés.

15. Kits d'analyse pour le dosage des médiateurs et de leurs dérivés, caractérisés en ce qu'ils renferment l'un au moins des dérivés selon l'une quelconque des revendications 1 à 9.

## Claims (Claims for the following Contracting State(s): AT, BE, CH, DE, DK, FR, GB, IT, LI, LU, NL, SE.)

1. The derivatives of biologically active molecules containing a primary amine function and a hydroxylated nucleus as well as their addition salts with mineral or organic acids, characterized in that they correspond to general formula I:
[R'R''N-A-B-O-CH₂-CO]_{N}R₁ (I)
in which
n represents an integer from 1 to 10;
A represents a linear or branched alkylene chain containing 1 to 5 carbon atoms;
B represents an aromatic nucleus containing functional unit 6 to 10 carbon atoms, if appropriate substituted, and optionally containing a heteroatom;
- R₁ represents an amine remainder, an alcohol remainder chosen from the optionally substituted phenols and the C₁-C₁₆ aliphatic alcohols;
and
R' and R'' represent an alkyl radical containing 1 to 5 carbon atoms or a hydrogen atom, as well as their addition salts with mineral or organic acids.

2. The derivatives of formula (I) as defined in claim 1, as well as their addition salts with mineral or organic acids, characterized in that B represents a phenyl or indole nucleus, optionally substituted by one or more halogen atoms.

3. The derivatives according to claim 2 as well as their addition salts with mineral or organic acids, characterized in that R₁ represents an NHR radical in which R represents a remainder of a diamine, a protein, an amino acid or a polypeptide constituted by at most 5 amino acids or derivatives of said amino acids or polypeptides.

4. The derivatives according to claim 1, 2 or 3, characterized in that R' and R'' represent a linear or branched alkyl radical containing 1 to 5 carbon atoms, and A represents a -CH₂-CH₂- radical, as well as their addition salts with mineral or organic acids.

5. One of the derivatives whose names follow:
- tryptamine-5-0-carboxymethylglycyltyrosinamide [S-CM-GTNH₂],
- tryptamine-5-0-carboxymethyltyrosylglycinamide
- as well as their addition salts with mineral or organic acids.

6. One of the derivatives whose names follow:
- tryptamine-5-0-carboxymethylglycyliodotyrosinamide [S-CM-iodoGTNH₂],
- tryptamine-5-0-carboxymethyliodotyrosylglycinamide
- as well as their addition salts with mineral or organic acids.

7. The derivatives of biologically active molecules containing a primary amine function and a hydroxylated nucleus as well as their addition salts with mineral or organic acids, characterized in that they correspond to formula:
R'R''N-A-B-O-CH₂-CO-NH-R-NH-CO-CH₂-O-B-A-N-R'R''
in which A, B, R' and R'' have the meaning indicated in claim 1, and R the meaning indicated in claim 10, or their amides.

8. The derivatives according to any one of claims 1 to 5, characterized in that they are presented in labelled form, the marker can be a radioactive element, in particular ¹²⁵I, a fluorescent molecule, an enzyme, a protein carrying a colloidal gold or a contrast medium.

9. The derivatives according to any one of claims 1 to 5, characterized in that they contain a protein grafted onto the O-carboxymethyl member according to the process of claim 10.

10. Preparation process for the derivatives of formula (I) as defined in claim 1 as well as their addition salts with mineral or organic acids, characterized in that a derivative of formula (II):
R'R''N-A-B-OH (II)
in which R', R'', A and B have the meaning already indicated, is reacted with a derivative containing a protector group of the amine functions in the case where R' = R''=H, in order to obtain a derivative of formula (III):
Y-HN-A-B-OH (III)
in which A and B have the meaning already indicated and Y represents an easily cleavable protector group of the amine functions, which is reacted with a halogenoacetic acid in order to obtain a derivative of formula (IV):
Y-HN-A-B-O-CH₂COOH (IV)
in which A, B and Y have the meaning already indicated, which is reacted with an amine derivative or an alcohol derivative in order to obtain the derivative of formula (I) as defined above, if appropriate, in the form of a dimer of formula (I'):
R'R''N-A-B-O-CH₂-CO-R-NH-NH-CO-CH₂-O-B-A-N-R'R'' (I')
in the case of a diamine, or a polymer in the case of a polyamine, R representing the corresponding diamine or polyamine remainder which is isolated and, if desired, salified.

11. Pharmaceutical compositions characterized in that they contain at least one of the medicaments as defined in one of claims 1 to 9 as active ingredient.

12. Use of the derivatives according to any one of claims 4 and 5 for the visualization of endogenous mediator binding sites.

13. Use of the derivatives according to any one of claims 5 to 8 for the purification of endogenous mediator receptors.

14. Use of the derivatives according to claim 9 for the represents preparation of antibodies directed against endoge mediators and their derivatives.

15. Analytical kits for determining mediators and their derivatives, characterized in that they contain at least one of the derivatives according to any one of claims 1 to 9.

## Claims (Claims for the following Contracting State(s): ES)

1. Preparation process in which a derivative of biologically active molecules containing a primary amine function and a hydroxylated nucleus or one of its addition salts with mineral or organic acids is prepared, characterized in that it corresponds to general formula I:
[R'R''N-A-B-O-CH₂-CO]_{N}R₁ (I)
in which
n represents an integer from 1 to 10;
A represents a linear or branched alkylene chain containing 1 to 5 carbon atoms;
B represents an aromatic nucleus containing 6 to 10 carbon atoms, if appropriate substituted, and optionally containing a heteroatom;
- R₁ represents an amine remainder, an alcohol remainder chosen from the optionally substituted phenols and the C₁-C₁₆ aliphatic alcohols;
and
R' and R'' represent an alkyl radical containing from 1 to 5 carbon atoms or a hydrogen atom, as well as their addition salts with mineral or organic acids, characterized in that a derivative of formula (II):
R'R''N-A-B-OH (II)
in which R', R'', A and B have the meaning already indicated, is reacted with a derivative containing a protector group of the amine functions in the case where R' = R''=H, in order to obtain a derivative of formula (III):
Y-HN-A-B-OH (III)
in which A and B have the meaning already indicated and Y represents an easily cleavable protector group of the amine functions, which is reacted with a halogenoacetic acid in order to obtain a derivative of formula (IV):
Y-HN-A-B-O-CH₂COOH (IV)
in which A, B and Y have the meaning already indicated, which is reacted with an amine derivative or an alcohol derivative in order to obtain the derivative of formula (I) as defined above, if appropriate, in the form of a dimer of formula (I'):
R'R''N-A-B-O-CH₂-CO-NH-R-NH-CO-CH₂-O-B-A-N-R'R'' (I')
in the case of a diamine, or in the form of a polymer in the case of a polyamine, R representing the corresponding diamine or polyamine remainder, which is isolated and, if desired, salified.

2. Process according to claim 1, characterized in that a derivative of formula (I) as defined in claim 1 or one of its addition salts with mineral or organic acids is prepared, in which B represents a phenyl or indole nucleus, optionally substituted by one or more halogen atoms.

3. Process according to claim 2 , characterized in that a derivative or one of its addition salts with mineral or organic acids is prepared, in which R₁ represents an NHR radical in which R represents a remainder of a diamine, a protein, an amino acid or a polypeptide constituted by at most 5 amino acids or derivatives of said amino acids or polypeptides.

4. Process according to claim 1, 2 or 3, characterized in that a derivative or one of its addition salts with mineral or organic acids is prepared, in which R' and R'' represent a linear or branched alkyl radical containing 1 to 5 carbon atoms, and A represents a -CH₂-CH₂- radical, as well as their addition salts with mineral or organic acids.

5. Preparation process according to claim 1 characterized in that one of the derivatives whose names follow is prepared:
- tryptamine-5-0-carboxymethylglycyltyrosinamide [S-CM-GTNH₂],
- tryptamine-5-0-carboxymethyltyrosylglycinamide
- or one of their addition salts with mineral or organic acids.

6. Preparation process according to claim 1 characterized in that one of the derivatives whose names follow is prepared:
- tryptamine-5-0-carboxymethylglycyliodotyrosinamide [S-CM-iodoGTNH₂],
- tryptamine-5-0-carboxymethyliodotyrosylglycinamide
- or one of their addition salts with mineral or organic acids.

7. Preparation process according to claim 1 characterized in that a derivative of biologically active molecules containing a primary amine function and a hydroxylated nucleus or one of its addition salts with mineral or organic acids is prepared, corresponding to the formula:
R'R''N-A-B-O-CH₂-CO-NH-R-NH-CO-CH₂-O-B-A-N-R'R''
in which A, B, R' and R'' have the meaning indicated in claim 1, and R the meaning indicated in claim 10, or one of its amides.

8. Preparation process characterized in that a derivative according to any one of claims 1 to 5 is prepared, presented in labelled form, the marker can be a radioactive element, in particular ¹²⁵I, a fluorescent molecule, an enzyme, a protein carrying a colloidal gold or a contrast medium.

9. Preparation process characterized in that a derivative according to any one of claims 1 to 5 is prepared, containing a protein grafted onto the O-carboxymethyl member according to the process of claim 1.

10. Preparation process for pharmaceutical compositions characterized in that the said compositions contain at least one of the medicaments as defined in one of claims 1 to 9 as active ingredient in which said active ingredient is mixed with a pharmaceutically acceptable excipient.

11. Use of the derivatives according to any one of claims 4 and 5 for the visualization of endogenous mediator binding sites.

12. Use of the derivatives according to any one of claims 5 to 8 for the purification of endogenous mediator receptors.

13. Use of the derivatives according to claim 9 for the preparation of antibodies directed against endogenous mediators and their derivatives.

14. Analytical kits for determining mediators and their derivatives, characterized in that they contain at least one of the derivatives according to any one of claims 1 to 9.

## Claims (Claims for the following Contracting State(s): GR)

1. The derivatives of biologically active molecules containing a primary amine function and a hydroxylated nucleus as well as their addition salts with mineral or organic acids, characterized in that they correspond to general formula I:
[R'R''N-A-B-O-CH₂-CO]_{N}R₁ (I)
in which
n represents an integer from 1 to 10;
A represents a linear or branched alkylene chain containing 1 to 5 carbon atoms;
B represents an aromatic nucleus containing functional unit 6 to 10 carbon atoms, if appropriate substituted, and optionally containing a heteroatom;
- R₁ represents an amine remainder, an alcohol remainder chosen from the optionally substituted phenols and the C₁-C₁₆ aliphatic alcohols;
and
R' and R'' represent an alkyl radical containing 1 to 5 carbon atoms or a hydrogen atom, as well as their addition salts with mineral or organic acids.

2. The derivatives of formula (I) as defined in claim 1, as well as their addition salts with mineral or organic acids, characterized in that B represents a phenyl or indole nucleus, optionally substituted by one or more halogen atoms.

3. The derivatives according to claim 2 as well as their addition salts with mineral or organic acids, characterized in that R₁ represents an NHR radical in which R represents a remainder of a diamine, a protein, an amino acid or a polypeptide constituted by at most 5 amino acids or derivatives of said amino acids or polypeptides.

4. The derivatives according to claim 1, 2 or 3, characterized in that R' and R'' represent a linear or branched alkyl radical containing 1 to 5 carbon atoms, and A represents a -CH₂-CH₂- radical, as well as their addition salts with mineral or organic acids.

5. One of the derivatives whose names follow:
- tryptamine-5-0-carboxymethylglycyltyrosinamide [S-CM-GTNH₂],
- tryptamine-5-0-carboxymethyltyrosylglycinamide
- as well as their addition salts with mineral or organic acids.

6. One of the derivatives whose names follow:
- tryptamine-5-0-carboxymethylglycyliodotyrosinamide [S-CM-iodoGTNH₂],
- tryptamine-5-0-carboxymethyliodotyrosylglycinamide
- as well as their addition salts with mineral or organic acids.

7. The derivatives of biologically active molecules containing a primary amine function and a hydroxylated nucleus as well as their addition salts with mineral or organic acids, characterized in that they correspond to formula:
R'R''N-A-B-O-CH₂-CO-NH-R-NH-CO-CH₂-O-B-A-N-R'R''
in which A, B, R' and R'' have the meaning indicated in claim 1, and R the meaning indicated in claim 10, or their amides.

8. The derivatives according to any one of claims 1 to 5, characterized in that they are presented in labelled form, the marker can be a radioactive element, in particular ¹²⁵I, a fluorescent molecule, an enzyme, a protein carrying a colloidal gold or a contrast medium.

9. The derivatives according to any one of claims 1 to 5, characterized in that they contain a protein grafted onto the O-carboxymethyl member according to the process of claim 10.

10. Preparation process for the derivatives of formula (I) as defined in claim 1 as well as their addition salts with mineral or organic acids, characterized in that a derivative of formula (II):
R'R''N-A-B-OH (II)
in which R', R'', A and B have the meaning already indicated, is reacted with a derivative containing a protector group of the amine functions in the case where R' = R''=H, in order to obtain a derivative of formula (III):
Y-HN-A-B-OH (III)
in which A and B have the meaning already indicated and Y represents an easily cleavable protector group of the amine functions, which is reacted with a halogenoacetic acid in order to obtain a derivative of formula (IV):
Y-HN-A-B-O-CH₂COOH (IV)
in which A, B and Y have the meaning already indicated, which is reacted with an amine derivative or an alcohol derivative in order to obtain the derivative of formula (I) as defined above, if appropriate, in the form of a dimer of formula (I'):
R'R''N-A-B-O-CH₂-CO-R-NH-NH-CO-CH₂-O-B-A-N-R'R'' (I')
in the case of a diamine, or a polymer in the case of a polyamine, R representing the corresponding diamine or polyamine remainder which is isolated and, if desired, salified.

11. Preparation process for pharmaceutical compositions characterized in that they contain at least one of the medicaments as defined in one of claims 1 to 9 as active ingredient, in which said active ingredient is mixed with a pharmaceutically acceptable excipient.

12. Use of the derivatives according to any one of claims 4 and 5 for the visualization of endogenous mediator binding sites.

13. Use of the derivatives according to any one of claims 5 to 8 for the purification of endogenous mediator receptors.

14. Use of the derivatives according to claim 9 for the preparation of antibodies directed against endogenous mediators and their derivatives.

15. Analytical kits for determining mediators and their derivatives, characterized in that they contain at least one of the derivatives according to any one of claims 1 to 9.

## Patentansprüche (Patentansprüche für folgende(n) Vertragsstaat(en): AT, BE, CH, DE, DK, FR, GB, IT, LI, LU, NL, SE)

1. Derivate biologisch aktiver Moleküle, die eine primäre Aminfunktion und einen Hydroxylkern umfassen, und ihre Additionssalze mit mineralischen oder organischen Säuren, dadurch gekennzeichnet, daß sie die allgemeine Formel (I) aufweisen:
[R'R''N-A-B-O-CH₂-CO]ₙR₁ (I),
worin n eine ganze Zahl von 1 bis 10 ist; A eine gerade oder verzweigte Alkylenkette mit 1 bis 5 Kohlenstoffatomen bedeutet; B einen aromatischen Kern mit 6 bis 10 Kohlenstoffatomen, die gegebenenfalls substituiert sind und gegebenenfalls ein Heteroatom umfassen, darstellt; R₁ ein Aminrest, ein Alkoholrest, ausgewählt aus gegebenenfalls substituierten Phenolen und aliphatischen C₁-C₁₆-Alkohlen, ist; und R' und R'' einen Alkylrest mit 1 bis 5 Kohlenstoffatomen oder einem Wasserstoffatom bedeuten; und ihre Additionssalze mit mineralischen oder organischen Säuren.

2. Derivate der Formel (I), wie in Anspruch 1 definiert, und ihre Additionssalze mit mineralischen oder organischen Säuren, dadurch gekennzeichnet, daß B einen Phenyl- oder Indolkern, gegebenenfalls substituiert durch ein oder mehrere Halogenatome, darstellt.

3. Derivate nach Anspruch 2 und ihre Additionssalze mit mineralischen oder organischen Säuren, dadurch gekennzeichnet, daß R₁ ein Rest NHR ist, worin R einen Diamin-, Protein-, Aminosäure- oder Polypeptidrest, der aus mehr als 5 Aminosäuren oder Derivaten der Aminosäuren oder Polypeptiden besteht, bedeutet.

4. Derivate nach Anspruch 1, 2 oder 3, dadurch gekennzeichnet, daR R' und R'' einen geraden oder verzweigten Alkylrest mit 1 bis 5 Kohlenstoffatomen bedeuten, und A einen Rest -CH₂-CH₂- darstellt, und ihre Additionssalze mit mineralischen oder organischen Säuren.

5. Eines der folgenden Derivate:
- Tryptamin-5-O-carboxymethylglycyltyrosinamid [S-CM-GTNH₂],
- Tryptamin-5-O-carboxymethyltyrosylglycinamid
- und ihre Additionssalze mit mineralischen oder organischen Säuren.

6. Eines der folgenden Derivate:
- Tryptamin-5-O-carboxymethylglycyljodtyrosinamid [S-CM-Jod-GTNH₂],
- Tryptamin-5-O-carboxymethyljodtyrosylglycinamid
- und ihre Additionssalze mit mineralischen oder organischen Säuren.

7. Derivate biologisch aktiver Moleküle, die eine primäre Aminfunktion und einen Hydroxylkern umfassen, und ihre Additionssalze mit mineralischen oder organischen Säuren, dadurch gekennzeichnet, daß sie die Formel aufweisen:
R'R''N-A-B-O-CH₂-CO-NH-R-NH-CO-CH₂-O-B-A-NR'R''
worin A, B, R' und R'' die in Anspruch 1 angegebene Bedeutung haben, und R die in Anspruch 10 angegebene, oder ihre Amide.

8. Derivate nach einem der Ansprüche 1 bis 5, dadurch gekennzeichnet, daß sie in markierter Form vorliegen, wobei ihr Marker ein radioaktives Element, insbesondere ¹²⁵J, ein fluoreszierendes Molekül, ein Enzym, ein kolloidales Gold tragendes Protein oder ein Kontrastmittel sein kann.

9. Derivate nach einem der Ansprüche 1 bis 5, dadurch gekennzeichnet, daß sie ein gemäß dem Verfahren nach Anspruch 10 in das O-Carboxymethyl-Kettenglied implantiertes Protein umfassen.

10. Verfahren zur Herstellung von Derivaten der Formel (I), wie in Anspruch 1 definiert, und ihrer Additionssalze mit mineralischen oder organischen Säuren, dadurch gekennzeichnet, daß ein Derivat der Formel (II):
R'R''N-A-B-OH (II),
worin R', R'', A und B die bereits angegebene Bedeutung haben, mit einem Derivat, das eine Schutzgruppe für die Amingruppen aufweist, wenn R' = R'' = H, umgesetzt wird, wobei ein Derivat der Formel (III):
Y-HN-A-B-OH (III),
worin A und B die bereits angegebene Bedeutung haben, und Y eine Schutzgruppe für die leicht abspaltbaren Amingruppen darstellt, erhalten wird, das mit einer Halogenessigsäure umgesetzt wird, wobei ein Derivat der Formel (IV):
Y-HN-A-B-O-CH₂COOH (IV),
worin A, B und Y die bereits angegebene Bedeutung haben, erhalten wird, das mit einem Amin-Derivat oder einen Alkohol-Derivat umgesetzt wird, wobei das Derivat der Formel (I), wie vorstehend definiert, erhalten wird, gegebenenfalls in dimerer Form der Formel (I'):
R'R''N-A-B-O-CH₂-CO-NH-R-NH-CO-CH₂-O-B-A-NR' R'' (I'),
wobei im Fall eines Diamins oder Polymers im Fall eines Polyamins R den Rest darstellt, der dem Diamin oder Polyamin entspricht, das isoliert und, wenn gewünscht, versalzt wird.

11. Pharmazeutische Zusammensetzungen, dadurch gekennzeichnet, daß sie als aktiven Bestandteil zumindest eines der Medikamente, wie in einem der Ansprüche 1 bis 9 definiert, umfassen.

12. Verwendung der Derivate nach einem der Ansprüche 4 und 5 bei der Darstellung von Bindungsstellen endogener Mediatoren.

13. Verwendung der Derivate nach einem der Ansprüche 5 bis 8 bei der Reinigung von Rezeptoren endogener Mediatoren.

14. Verwendung der Derivate nach Anspruch 9 bei der Herstellung von Antikörpern gegen endogene Mediatoren und ihre Derivate.

15. Analyseausrüstungen zur Dosierung von Mediatoren und ihren Derivaten, dadurch gekennzeichnet, daß sie zumindest eines der Derivate nach einem der Ansprüche 1 bis 9 umfassen.

## Patentansprüche (Patentansprüche für folgende(n) Vertragsstaat(en): ES)

1. Herstellungsverfahren, bei welchem ein Derivat biologisch aktiver Moleküle, die eine primäre Aminfunktion und einen Hydroxylkern umfassen, oder eines seiner Additionssalze mit mineralischen oder organischen Säuren, dadurch gekennzeichnet, daß es die allgemeine Formel (I) aufweist:
[R'R''N-A-B-O-CH₂-CO]ₙR₁ (I),
worin n eine ganze Zahl von 1 bis 10 ist; A eine gerade oder verzweigte Alkylenkette mit 1 bis 5 Kohlenstoffatomen bedeutet; B einen aromatischen Kern mit 6 bis 10 Kohlenstoffatomen, die gegebenenfalls substituiert sind und gegebenenfalls ein Heteroatom umfassen, darstellt; R₁ ein Aminrest, ein Alkoholrest, ausgewählt aus gegebenenfalls substituierten Phenolen und aliphatischen C₁-C₁₆-Alkohlen, ist; und R' und R'' einen Alkylrest mit 1 bis 5 Kohlenstoffatomen oder einem Wasserstoffatom bedeuten; und ihre Additionssalze mit mineralischen oder organischen Säuren hergestellt werden; dadurch gekennzeichnet, daß ein Derivat der Formel (II):
R'R''N-A-B-OH (II),
worin R', R'', A und B die bereits angegebene Bedeutung haben, mit einem Derivat, das eine Schutzgruppe für die Amingruppen aufweist, wenn R' = R'' = H, umgesetzt wird, wobei ein Derivat der Formel (III):
Y-HN-A-B-OH (III),
worin A und B die bereits angegebene Bedeutung haben, und Y eine Schutzgruppe für die leicht abspaltbaren Amingruppen darstellt, erhalten wird, das mit einer Halogenessigsäure umgesetzt wird, wobei ein Derivat der Formel (IV):
Y-HN-A-B-O-CH₂COOH (IV),
worin A, B und Y die bereits angegebene Bedeutung haben, erhalten wird, das mit einem Amin-Derivat oder einen Alkohol-Derivat umgesetzt wird, wobei das Derivat der Formel (I), wie vorstehend definiert, erhalten wird, gegebenenfalls in dimerer Form der Formel (I'):
R'R''N-A-B-O-CH₂-CO-NH-R-NH-CO-CH₂-O-B-A-NR'R'' (I'),
wobei im Fall eines Diamins oder Polymers im Fall eines Polyamins R den Rest darstellt, der dem Diamin oder Polyamin entspricht, das isoliert und, wenn gewünscht, versalzt wird.

2. Verfahren nach Anspruch 1, dadurch gekennzeichnet, daß ein Derivat der Formel (I), wie in Anspruch 1 definiert, oder eines seiner Additionssalze mit mineralischen oder organischen Säuren hergestellt wird, wobei B einen Phenyl- oder Indolkern, gegebenenfalls substituiert durch ein oder mehrere Halogenatome, darstellt.

3. Verfahren nach Anspruch 2, dadurch gekennzeichnet, daß ein Derivat oder eines seiner Additionssalze mit mineralischen oder organischen Säuren hergestellt wird, worin R₁ ein Rest NHR ist, wobei R einen Diamin-, Protein-, Aminosäure- oder Polypeptidrest, der aus mehr als 5 Aminosäuren oder Derivaten der Aminosäuren oder Polypeptiden besteht, bedeutet.

4. Verfahren nach Anspruch 1, 2 oder 3, dadurch gekennzeichnet, daß ein Derivat oder eines seiner Additionssalze mit mineralischen oder organischen Säuren hergestellt wird, worin R' und R'' einen geraden oder verzweigten Alkylrest mit 1 bis 5 Kohlenstoffatomen bedeuten, und A einen Rest -CH₂-CH₂- darstellt.

5. Herstellungsverfahren nach Anspruch 1, dadurch gekennzeichnet, daß eines der folgenden Derivate:
- Tryptamin-5-O-carboxymethylglycyltyrosinamid [S-CM-GTNH₂],
- Tryptamin-5-O-carboxymethyltyrosylglycinamid
- oder eines seiner Additionssalze mit mineralischen oder organischen Säuren hergestellt wird.

6. Herstellungsverfahren nach Anspruch 1, dadurch gekennzeichnet, daß eines der folgenden Derivate:
- Tryptamin-5-O-carboxymethylglycyljodtyrosinamid [S-CM-Jod-GTNH₂],
- Tryptamin-5-O-carboxymethyljodtyrosylglycinamid
- oder eines seiner Additionssalze mit mineralischen oder organischen Säuren hergestellt wird.

7. Herstellungsverfahren nach Anspruch 1, dadurch gekennzeichnet, daß ein Derivat biologisch aktiver Moleküle, die eine primäre Aminfunktion und einen Hydroxylkern umfassen, oder eines seiner Additionssalze mit mineralischen oder organischen Säuren der Formel:
R' R''N-A-B-O-CH₂-CO-NH-R-NH-CO-CH₂-O-B-A-NR'R''
worin A, B, R' und R'' die in Anspruch 1 angegebene Bedeutung haben, und R die in Anspruch 1 angegebene, oder eines seiner Amide hergestellt wird.

8. Herstellungsverfahren, dadurch gekennzeichnet, daß ein Derivat nach einem der Ansprüche 1 bis 5 hergestellt wird, das in markierter Form vorliegt, wobei der Marker ein radioaktives Element, insbesondere ¹²⁵J, ein fluoreszierendes Molekül, ein Enzym, ein kolloidales Gold tragendes Protein oder ein Kontrastmittel sein kann.

9. Herstellungsverfahren, dadurch gekennzeichnet, daß ein Derivat nach einem der Ansprüche 1 bis 5 hergestellt wird, das ein gemäß dem verfahren nach Anspruch 1 in das O-Carboxymethyl-Kettenglied implantiertes Protein umfaßt.

10. Verfahren zur Herstellung pharmazeutischer Zusammensetzungen, dadurch gekennzeichnet, daß die Zusammensetzungen als aktiven Bestandteil zumindest eines der Medikamente, wie in einem der Ansprüche 1 bis 9 definiert, umfassen, bei welchem der aktive Bestandteil mit einem pharmazeutisch annehmbaren Exzipienten gemischt wird.

11. Verwendung der Derivate, hergestellt nach einem der Ansprüche 4 und 5, bei der Darstellung von Bindungsstellen endogener Mediatoren.

12. Verwendung der Derivate, hergestellt nach einem der Ansprüche 5 bis 8, bei der Reinigung von Rezeptoren endogener Mediatoren.

13. Verwendung der Derivate, hergestellt nach Anspruch 9, bei der Herstellung von Antikörpern gegen endogene Mediatoren und ihre Derivate.

14. Analyseausrüstungen zur Dosierung von Mediatoren und ihren Derivaten, dadurch gekennzeichnet, daß sie zumindest eines der Derivate, hergestellt nach einem der Ansprüche 1 bis 9, umfassen.

## Patentansprüche (Patentansprüche für folgende(n) Vertragsstaat(en): GR)

1. Derivate biologisch aktiver Moleküle, die eine primäre Aminfunktion und einen Hydroxylkern umfassen, und ihre Additionssalze mit mineralischen oder organischen Säuren, dadurch gekennzeichnet, daß sie die allgemeine Formel (I) aufweisen:
[R'R''N-A-B-O-CH₂-CO]ₙR₁ (I),
worin n eine ganze Zahl von 1 bis 10 ist; A eine gerade oder verzweigte Alkylenkette mit 1 bis 5 Kohlenstoffatomen bedeutet; B einen aromatischen Kern mit 6 bis 10 Kohlenstoffatomen, die gegebenenfalls substituiert sind und gegebenenfalls ein Heteroatom umfassen, darstellt; R₁ ein Aminrest, ein Alkoholrest, ausgewählt aus gegebenenfalls substituierten Phenolen und aliphatischen C₁-C₁₆-Alkohlen, ist; und R' und R'' einen Alkylrest mit 1 bis 5 Kohlenstoffatomen oder einem Wasserstoffatom bedeuten; und ihre Additionssalze mit mineralischen oder organischen Säuren.

2. Derivate der Formel (I), wie in Anspruch 1 definiert, und ihre Additionssalze mit mineralischen oder organischen Säuren, dadurch gekennzeichnet, daß B einen Phenyl- oder Indolkern, gegebenenfalls substituiert durch ein oder mehrere Halogenatome, darstellt.

3. Derivate nach Anspruch 2 und ihre Additionssalze mit mineralischen oder organischen Säuren, dadurch gekennzeichnet, daß R₁ ein Rest NHR ist, worin R einen Diamin-, Protein-, Aminosäure- oder Polypeptidrest, der aus mehr als 5 Aminosäuren oder Derivaten der Aminosäuren oder Polypeptiden besteht, bedeutet.

4. Derivate nach Anspruch 1, 2 oder 3, dadurch gekennzeichnet, daß R' und R'' einen geraden oder verzweigten Alkylrest mit 1 bis 5 Kohlenstoffatomen bedeuten, und A einen Rest -CH₂-CH₂- darstellt, und ihre Additionssalze mit mineralischen oder organischen Säuren.

5. Eines der folgenden Derivate:
- Tryptamin-5-O-carboxymethylglycyltyrosinamid [S-CM-GTNH₂],
- Tryptamin-5-O-carboxymethyltyrosylglycinamid
- und ihre Additionssalze mit mineralischen oder organischen Säuren.

6. Eines der folgenden Derivate:
- Tryptamin-5-O-carboxymethylglycyljodtyrosinamid [S-CM-Jod-GTNH₂],
- Tryptamin-5-O-carboxymethyljodtyrosylglycinamid
- und ihre Additionssalze mit mineralischen oder organischen Säuren.

7. Derivate biologisch aktiver Moleküle, die eine primäre Aminfunktion und einen Hydroxylkern umfassen, und ihre Additionssalze mit mineralischen oder organischen Säuren, dadurch gekennzeichnet, daß sie die Formel aufweisen:
R' R''N-A-B-O-CH₂-CO-NH-R-NH-CO-CH₂-O-B-A-NR'R''
worin A, B, R' und R'' die in Anspruch 1 angegebene Bedeutung haben, und R die in Anspruch 10 angegebene, oder ihre Amide.

8. Derivate nach einem der Ansprüche 1 bis 5, dadurch gekennzeichnet, daß sie in markierter Form vorliegen, wobei ihr Marker ein radioaktives Element, insbesondere ¹²⁵J, ein fluoreszierendes Molekül, ein Enzym, ein kolloidales Gold tragendes Protein oder ein Kontrastmittel sein kann.

9. Derivate nach einem der Ansprüche 1 bis 5, dadurch gekennzeichnet, daß sie ein gemäß dem Verfahren nach Anspruch 10 in das O-Carboxymethyl-Kettenglied implantiertes Protein umfassen.

10. Verfahren zur Herstellung von Derivaten der Formel (I), wie in Anspruch 1 definiert, und ihrer Additionssalze mit mineralischen oder organischen Säuren, dadurch gekennzeichnet, daß ein Derivat der Formel (II):
R'R''N-A-B-OH (II),
worin R', R'', A und B die bereits angegebene Bedeutung haben, mit einem Derivat, das eine Schutzgruppe für die Amingruppen aufweist, wenn R' = R'' = H, umgesetzt wird, wobei ein Derivat der Formel (III):
Y-HN-A-B-OH (III),
worin A und B die bereits angegebene Bedeutung haben, und Y eine Schutzgruppe für die leicht abspaltbaren Amingruppen darstellt, erhalten wird, das mit einer Halogenessigsäure umgesetzt wird, wobei ein Derivat der Formel (IV):
Y-HN-A-B-O-CH₂COOH (IV),
worin A, B und Y die bereits angegebene Bedeutung haben, erhalten wird, das mit einem Amin-Derivat oder einen Alkohol-Derivat umgesetzt wird, wobei das Derivat der Formel (I), wie vorstehend definiert, erhalten wird, gegebenenfalls in dimerer Form der Formel (I'):
R'R''N-A-B-O-CH₂-CO-NH-R-NH-CO-CH₂-O-B-A-NR'R'' (I'),
wobei im Fall eines Diamins oder Polymers im Fall eines Polyamins R den Rest darstellt, der dem Diamin oder Polyamin entspricht, das isoliert und, wenn gewünscht, versalzt wird.

11. Verfahren zur Herstellung pharmazeutischer Zusammensetzungen, dadurch gekennzeichnet, daß sie als aktiven Bestandteil zumindest eines der Medikamente, wie in einem der Ansprüche 1 bis 9 definiert, umfassen, bei welchem der aktive Bestandteil mit einem pharmazeutisch annehmbaren Exzipienten gemischt wird.

12. Verwendung der Derivate nach einem der Ansprüche 4 und 5 bei der Darstellung von Bindungsstellen endogener Mediatoren.

13. Verwendung der Derivate nach einem der Ansprüche 5 bis 8 bei der Reinigung von Rezeptoren endogener Mediatoren.

14. Verwendung der Derivate nach Anspruch 9 bei der Herstellung von Antikörpern gegen endogene Mediatoren und ihre Derivate.

15. Analyseausrüstungen zur Dosierung von Mediatoren und ihren Derivaten, dadurch gekennzeichnet, daß sie zumindest eines der Derivate nach einem der Ansprüche 1 bis 9 umfassen.
